# EUROPEAN PATENT APPLICATION

(11) **EP 4 696 704 A1**
(43) Date of publication of application: **18.02.2026**
(21) Application number: 24788121.2
(22) Date of filing: 10.04.2024
(51) Int. Cl.: C07K 14/145, C12N 15/867, C12N 5/10, A61K 48/00

(54) **MUTANT VSVG AND TARGETING VECTOR**

(30) Priority: 10.04.2023 CN 202310375327
(71) Applicant: Shenzhen Genocury Biotech Co., Ltd., Shenzhen, Guangdong 518122 (CN)
(72) Inventor: HUANG, Ke, Shenzhen, Guangdong 518100 (CN); LI, Yuhang, Shenzhen, Guangdong 518100 (CN); ZHANG, Tian, Shenzhen, Guangdong 518100 (CN); LI, Jinbing, Shenzhen, Guangdong 518100 (CN)
(74) Representative: Winter, Brandl - Partnerschaft mbB
(86) International application number: PCT/CN2024/087105
(87) International publication number: WO 2024/213019

(57) **Abstract**

Provided are a mutant VSVG and a targeting vector, belonging to the field of vector delivery. An envelope glycoprotein of vesicular stomatitis virus having a first mutation and a second mutation is provided, the first mutation causes the envelope glycoprotein to be unable to mediate complement inactivation; and the second mutation weakens the ability of the envelope glycoprotein to be recognized by a receptor. A targeting vector is also provided, which comprises : a first molecule that binds to an endocytic receptor of a target cell and a second molecule that is non-complement inactivated and promotes the release of a substance carried by the targeting vector into the cytoplasm; the second molecule promotes the endosomal escape or lysosomal escape of the targeting vector and prevents the targeting vector from being inactivated by a complement.

## Description

### Cross-Reference to Related Applications

The disclosure claims the priority of the Chinese patent application with the application number of 2023103753276 and the invention name of "Mutant VSVG and targeting vector", which was submitted to the China National Intellectual Property Administration on April 10, 2023. The contents of these applications are incorporated herein by reference in their entirety.

### Reference to Sequence Listing Submitted in Electronic Form

The disclosure includes a sequence listing in XML format submitted in electronic form, which is incorporated herein by reference in its entirety. This sequence listing was created on April 10, 2024, and is named "JYSW-PA-PCT-NO-06-segl.xml".

### TECHNICAL FIELD

The disclosure belongs to the field of vector delivery, specifically to a mutant VSVG and targeting vector.

### BACKGROUND ART

The complement system is composed of a series of proteins, belonging to a part of the innate immune system. Complement exists in the serum, tissue fluid, and on the surface of cell membranes of normal humans and animals. After activation, complement exhibits enzymatic activity and are capable of undergoing intricate cascade reactions. The complement system is initiated through a series of sequential enzymatic cleavages among its components, ultimately forming pore-like membrane attack complexes on target microorganisms, which cause the microorganisms to rupture and die. Complement components can be activated by antigen-antibody complexes or antibodies. Once complement components are activated, they clear immune complexes through processes such as cytolysis, opsonization, phagocytosis, and the mediation of inflammatory responses, exhibiting corresponding biological functions. Complement is extensively involved in the defensive reaction and immunoregulation of the organism antimicrobial infection, at the same time, it also mediates immunopathological damage reactions, it is an effector system and an effector amplification system with important biological roles in the body.

Complement components with regulatory functions exist in soluble or membrane-bound forms, which mainly include: properdin (factor P), C1 inhibitor (C1INH), factor I, factor H, C4 binding protein (C4BP), S protein, SP40/40, membrane cofactor protein (MCP), decay accelerating factor (DAF), homologous restriction factor (HRF), and membrane inhibitor of reactive lysis (MIRL).

Currently, vectors based on the VSVG envelope glycoprotein have been widely used in clinical therapy, such as in chimeric antigen receptor T cell therapy (CAR-T), CAR-T cells are prepared by infecting T cells with VSVG-carrying lentiviruses, which encapsulate polynucleotides encoding CAR molecules. However, after the VSVG viral vector enters the serum, it will be recognized and inactivated by the complement, making it difficult for them to effectively reach target cells and exert their functions. Therefore, it cannot be directly used in in-vivo therapy based on intravenous injection. This is also a common problem faced by other targeted vectors.

Thus, it is necessary to modify existing delivery vectors to prevent them from being inactivated by complement, thereby improving the effectiveness of their delivery.

### SUMMARY

In one aspect, the disclosure provides an envelope glycoprotein of vesicular stomatitis virus with a first mutation and a second mutation, the first mutation causes the envelope glycoprotein to be unable to mediate complement inactivation; the second mutation weakens the ability of the envelope glycoprotein to be recognized by a receptor.

In a specific embodiment, the first mutation is that the extracellular domain of the envelope glycoprotein comprises the amino acid sequence as set forth in SEQ ID NO: 1 or an amino acid sequence having at least 50% identity to the amino acid sequence as set forth in SEQ ID NO: 1, and the amino acid sequence comprises at least one mutated amino acid as following:
a) the amino acid at position 214 of SEQ ID NO: 1;
b) the amino acid located at position 214 corresponding to SEQ ID NO: 1 by best global alignment;
c) the amino acid at position 352 of SEQ ID NO: 1;
d) the amino acid located at position 352 corresponding to SEQ ID NO: 1 by best global alignment;
e) the amino acid at position 50 of SEQ ID NO: 1;
f) the amino acid located at position 50 corresponding to SEQ ID NO: 1 by best global alignment;
g) the amino acid at position 146 of SEQ ID NO: 1;
h) the amino acid located at position 146 corresponding to SEQ ID NO: 1 by best global alignment.

The extracellular domain of the envelope glycoprotein comprises of vesicular stomatitis virus Indiana serotype (Indiana-VSVG) comprises the amino acid sequence as set forth in SEQ ID NO: 1.

In a specific embodiment, the mutation is an insertion, deletion or substitution.

In a specific embodiment, the amino acid sequence comprises at least one substituted amino acid as following:
a) the amino acid at position 214 of SEQ ID NO: 1;
b) the amino acid located at position 214 corresponding to SEQ ID NO: 1 by best global alignment;
c) the amino acid at position 352 of SEQ ID NO: 1;
d) the amino acid located at position 352 corresponding to SEQ ID NO: 1 by best global alignment;
e) the amino acid at position 50 of SEQ ID NO: 1;
f) the amino acid located at position 50 corresponding to SEQ ID NO: 1 by best global alignment;
g) the amino acid at position 146 of SEQ ID NO: 1;
h) the amino acid located at position 146 corresponding to SEQ ID NO: 1 by best global alignment.

In a specific embodiment, the amino acid sequence comprises one or more mutated amino acid as following: substitution of the amino acid at position T214, substitution of the amino acid at position T352, substitution of the amino acid at position K50, substitution of the amino acid at position S146.

In a specific embodiment, the amino acid sequence comprises one or more mutated amino acid as following: the amino acid at position 214 is substituted from threonine (T) to asparagine (N), and/or the amino acid at position 352 is substituted from threonine (T) to alanine (A), and/or the amino acid at position 50 is substituted from lysine (K) to threonine (T), and/or the amino acid at position 146 is substituted from serine (S) to threonine (T).

In a specific embodiment, the amino acid sequence comprises one of the mutated amino acid combinations as following:
(1) the amino acid at position 214 is substituted from threonine (T) to asparagine (N) and the amino acid at position 352 is substituted from threonine (T) to alanine (A);
(2) the amino acid at position 214 is substituted from threonine (T) to asparagine (N), the amino acid at position 352 is substituted from threonine (T) to alanine (A), the amino acid at position 50 is substituted from lysine (K) to threonine (T) and the amino acid at position 146 is substituted from serine (S) to threonine (T).

In a specific embodiment, the amino acid sequence having at least 50% identity to the amino acid sequence as set forth in SEQ ID NO: 1, the extracellular domain of the envelope glycoprotein of Cocal virus comprises the amino acid sequence as set forth in SEQ ID NO: 10:

KFSIVFPQSQKGNWKNVPSSYHYCPSSSDQNWHNDLLGITMKVKMP
KTHKAIQADGWMCHAAKWITTCDFRWYGPKYITHSIHSIQPTSEQCKESIKQT
KQGTWMSPGFPPQNCGYATVTDSVAVVVQATPHHVLVDEYTGEWIDSQFPNG
KCETEECETVHNSTVWYSDYKVTGLCDATLVDTEITFFSEDGKKESIGKPNTG
YRSNYFAYEKGDKVCKMNYCKHAGVRLPSGVWFEFVDQDVYAAAKLPECP
VGATISAPTQTSVDVSLILDVERILDYSLCQETWSKIRSKQPVSPVDLSYLAPK
NPGTGPAFTIINGTLKYFETRYIRIDIDNPIISKMVGKISGSQTERELWTEWFPYE
GVEIGPNGILKTPTGYKFPLFMIGHGMLDSDLHKTSQAEVFEHPHLAEAPKQL
PEEETLFFGDTGISKNPVELIEGWFSSWKSTVVTFFFAIGVFILLYVVARIVIAVR
YRYQGSNNKRIYNDIEMSRFRK.

Cocal virus, i.e., vesicular stomatitis virus Cocal serotype, the extracellular domain of the envelope glycoprotein of V vesicular stomatitis virus Cocal serotype (Cocal-VSVG) comprises the amino acid sequence as set forth in SEQ ID NO: 10; the amino acid sequence as set forth in SEQ ID NO: 10 has at least 50% identity with the amino acid sequence as set forth in SEQ ID NO: 1, i.e., the extracellular domain of Indiana-VSVG.

In a specific embodiment, SEQ ID NO: 10 comprises at least one mutated amino acid as following:
a) the amino acid at position 214 of SEQ ID NO: 10;
b) the amino acid located at position 214 corresponding to SEQ ID NO: 10 by best global alignment;
c) the amino acid at position 352 of SEQ ID NO: 10;
d) the amino acid located at position 352 corresponding to SEQ ID NO: 10 by best global alignment;
e) the amino acid at position 50 of SEQ ID NO: 10;
f) the amino acid located at position 50 corresponding to SEQ ID NO: 10 by best global alignment;
g) the amino acid at position 146 of SEQ ID NO: 10;
h) the amino acid located at position 146 corresponding to SEQ ID NO: 10 by best global alignment.

In a specific embodiment, the mutation is an insertion, deletion or substitution.

In a specific embodiment, SEQ ID NO: 10 comprises at least one substituted amino acid as following:
a) the amino acid at position 214 of SEQ ID NO: 10;
b) the amino acid located at position 214 corresponding to SEQ ID NO: 10 by best global alignment;
c) the amino acid at position 352 of SEQ ID NO: 10;
d) the amino acid located at position 352 corresponding to SEQ ID NO: 10 by best global alignment;
e) the amino acid at position 50 of SEQ ID NO: 10;
f) the amino acid located at position 50 corresponding to SEQ ID NO: 10 by best global alignment;
g) the amino acid at position 146 of SEQ ID NO: 10;
h) the amino acid located at position 146 corresponding to SEQ ID NO: 10 by best global alignment.

In a specific embodiment, SEQ ID NO: 10 comprises one or more mutated amino acid as following: substitution of the amino acid at position K214, substitution of the amino acid at position T352, substitution of the amino acid at position K50, substitution of the amino acid at position S146.

In a specific embodiment, SEQ ID NO: 10 comprises one or more mutated amino acid as following: the amino acid at position 214 is substituted from lysine (K) to asparagine (N), and/or the amino acid at position 352 is substituted from threonine (T) to alanine (A), and/or the amino acid at position 50 is substituted from lysine (K) to threonine (T), and/or the amino acid at position 146 is substituted from serine (S) to threonine (T).

In a specific embodiment, SEQ ID NO: 10 comprises one of the mutated amino acid combinations as following:
(1) substitution of the amino acid at position K214 and T352;
(2) substitution of the amino acid at position K214, T352, K50, and S 146.

In a specific embodiment, SEQ ID NO: 10 comprises one of the mutated amino acid combinations as following:
the amino acid at position 214 is substituted from lysine (K) to asparagine (N) and the amino acid at position 352 is substituted from threonine (T) to alanine (A);
the amino acid at position 214 is substituted from lysine (K) to asparagine (N), the amino acid at position 352 is substituted from threonine (T) to alanine (A), the amino acid at position 50 is substituted from lysine (K) to threonine (T) and the amino acid at position 146 is substituted from serine (S) to threonine (T).

In a specific embodiment of the envelope glycoprotein of vesicular stomatitis virus with a first mutation and a second mutation, any one of the first mutation enhances the ability of the envelope glycoprotein to counteract complement inactivation relative to before any one of the first mutations occurs; the second mutation weakens the ability of the envelope glycoprotein to be recognized by the receptor relative to before the occurrence of the second mutation.

In a specific embodiment, any one of the first mutation enhances the ability of the envelope glycoprotein to counteract complement inactivation relative to before any one of the first mutations occurs.

In a specific embodiment, the receptor is an envelope glycoprotein receptor.

In a specific embodiment, the envelope glycoprotein receptor is low-density lipoprotein receptor (LDL-R).

In a specific embodiment, the second mutation is that the extracellular domain of the envelope glycoprotein comprises the amino acid sequence as set forth in SEQ ID NO: 1 or an amino acid sequence having at least 50% identity to the amino acid sequence as set forth in SEQ ID NO: 1, and the amino acid sequence comprises at least one mutated amino acid as following:
1) substitution/deletion of the amino acid at position H8, substitution/deletion of the amino acid at position N9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position S183, substitution/deletion of the amino acid at position S179, substitution/deletion of the amino acid at position N180, substitution/deletion of the amino acid at position I182, substitution/deletion of the amino acid at position M184, substitution/deletion of the amino acid at position Y209, substitution/deletion of the amino acid at position I347, substitution/deletion of the amino acid at position T350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 of SEQ ID NO: 1;
2) substitution/deletion of the amino acid at position H8, substitution/deletion of the amino acid at position N9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position S183, substitution/deletion of the amino acid at position S179, substitution/deletion of the amino acid at position N180, substitution/deletion of the amino acid at position I182, substitution/deletion of the amino acid at position M184, substitution/deletion of the amino acid at position Y209, substitution/deletion of the amino acid at position I347, substitution/deletion of the amino acid at position T350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 corresponding to SEQ ID NO: 1 by best global alignment.

In a specific embodiment, the amino acid sequence comprises at least one mutated amino acid as following:
1) substitution of the amino acid at position H8, substitution of the amino acid at position N9, substitution of the amino acid at position Q10, substitution of the amino acid at position K47, deletion of the amino acid at position K47, substitution of the amino acid at position K50, substitution of the amino acid at position A51, substitution of the amino acid at position S183; substitution of the amino acid at position S179; substitution of the amino acid at position N180, substitution of the amino acid at position I182, substitution of the amino acid at position M184, substitution of the amino acid at position Y209, substitution of the amino acid at position I347, substitution of the amino acid at position T350, substitution of the amino acid at position T352, substitution of the amino acid at position E353, substitution of the amino acid at position R354, deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 of SEQ ID NO: 1;
2) substitution of the amino acid at position H8, substitution of the amino acid at position N9, substitution of the amino acid at position Q10, substitution of the amino acid at position K47, deletion of the amino acid at position K47, substitution of the amino acid at position K50, substitution of the amino acid at position A51, substitution of the amino acid at position S183; substitution of the amino acid at position S179; substitution of the amino acid at position N180, substitution of the amino acid at position I182, substitution of the amino acid at position M184, substitution of the amino acid at position Y209, substitution of the amino acid at position I347, substitution of the amino acid at position T350, substitution of the amino acid at position T352, substitution of the amino acid at position E353, substitution of the amino acid at position R354, deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 corresponding to SEQ ID NO: 1 by best global alignment.

In a specific embodiment, the amino acid sequence comprises one or more mutated amino acid as following: substitution/deletion of the amino acid at position K47, substitution/deletion of the amino acid at position R354.

In a specific embodiment, the amino acid sequence comprises one or more mutated amino acid as following: the amino acid at position 47 is substituted from lysine (K) to glutamine (Q); the amino acid at position 354 is substituted from arginine (R) to glutamine (Q), deletion of the amino acid at position 47, deletion of the amino acid at position 354.

In a specific embodiment, the amino acid sequence comprises one of the mutated amino acid as following:
(1) substitution of the amino acid at position K47;
(2) substitution of the amino acid at position R354.

In a specific embodiment, the amino acid sequence comprises one of the mutated amino acid as following:
(1) the amino acid at position 47 is substituted from lysine (K) to glutamine (Q);
(2) the amino acid at position 354 is substituted from arginine (R) to glutamine (Q).

In a specific embodiment, the amino acid sequence comprises at least one mutated amino acid as following:
deletion of amino acids at position 331-364, deletion of amino acids at position 344-354, substitution of the amino acid at position K47, deletion of the amino acid at position K47, substitution of the amino acid at position R354, deletion of the amino acid at position R354 of SEQ ID NO: 1;
deletion of amino acids at position 331-364, deletion of amino acids at position 344-354, substitution of the amino acid at position K47, deletion of the amino acid at position K47, substitution of the amino acid at position R354, deletion of the amino acid at position R354 corresponding to SEQ ID NO: 1 by best global alignment.

In a specific embodiment, the amino acid sequence comprises at least one mutated amino acid as following:
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 of SEQ ID NO: 1; and
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 corresponding to SEQ ID NO: 1 by best global alignment.

In a specific embodiment, the amino acid sequence comprises one of the mutated amino acid as following:
deletion of the amino acid at position K47 of SEQ ID NO: 1;
deletion of the amino acid at position K47 corresponding to SEQ ID NO: 1 by best global alignment;

In a specific embodiment, the second mutation is an amino acid sequence having at least 50% identity to the amino acid sequence as set forth in SEQ ID NO: 1, SEQ ID NO: 10 comprises at least one mutated amino acid as following:
substitution/deletion of the amino acid at position Q8, substitution/deletion of the amino acid at position S9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position D183, substitution/deletion of the amino acid at position A179, substitution/deletion of the amino acid at position T180, substitution/deletion of the amino acid at position V182, substitution/deletion of the amino acid at position T184, substitution/deletion of the amino acid at position Y209, substitution/deletion of the amino acid at position I347, substitution/deletion of the amino acid at position S350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 of SEQ ID NO: 10;
substitution/deletion of the amino acid at position Q8, substitution/deletion of the amino acid at position S9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position D183, substitution/deletion of the amino acid at position A179, substitution/deletion of the amino acid at position T180, substitution/deletion of the amino acid at position V182, substitution/deletion of the amino acid at position T184, substitution/deletion of the amino acid at position Y209, substitution/deletion of the amino acid at position I347, substitution/deletion of the amino acid at position S350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 corresponding to SEQ ID NO: 10 by best global alignment.

In a specific embodiment, SEQ ID NO: 10 comprises at least one mutated amino acid as following:
substitution of the amino acid at position Q8, substitution of the amino acid at position S9, substitution of the amino acid at position Q10, substitution or deletion of the amino acid at position K47, substitution of the amino acid at position K50, substitution of the amino acid at position A51, substitution of the amino acid at position D183, substitution of the amino acid at position A179, substitution of the amino acid at position T180, substitution of the amino acid at position V182, substitution of the amino acid at position T184, substitution of the amino acid at position Y209, substitution of the amino acid at position I347, substitution of the amino acid at position S350, substitution of the amino acid at position T352, substitution of the amino acid at position E353, substitution or deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids 345-353 of SEQ ID NO: 10;
substitution of the amino acid at position Q8, substitution of the amino acid at position S9, substitution of the amino acid at position Q10, substitution or deletion of the amino acid at position K47, substitution of the amino acid at position K50, substitution of the amino acid at position A51, substitution of the amino acid at position D183, substitution of the amino acid at position A179, substitution of the amino acid at position T180, substitution of the amino acid at position V182, substitution of the amino acid at position T184, substitution of the amino acid at position Y209, substitution of the amino acid at position I347, substitution of the amino acid at position S350, substitution of the amino acid at position T352, substitution of the amino acid at position E353, substitution or deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids 345-353 corresponding to SEQ ID NO: 10 by best global alignment.

In a specific embodiment, SEQ ID NO: 10 comprises at least one mutated amino acid as following:
deletion of amino acids at position 331-364, deletion of amino acids at position 344-354, substitution of the amino acid at position K47, deletion of the amino acid at position K47, substitution of the amino acid at position R354, deletion of the amino acid at position R354 of SEQ ID NO:10;
deletion of amino acids at position 331-364, deletion of amino acids at position 344-354, substitution of the amino acid at position K47, deletion of the amino acid at position K47, substitution of the amino acid at position R354, deletion of the amino acid at position R354 corresponding to SEQ ID NO: 10 by best global alignment.

In a specific embodiment, SEQ ID NO: 10 comprises at least one mutated amino acid as following:
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 of SEQ ID NO:10;and
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 corresponding to SEQ ID NO: 10 by best global alignment.

In a specific embodiment, SEQ ID NO: 10 comprises one of the mutated amino acid as following:
deletion of the amino acid at position K47 of SEQ ID NO:10;
deletion of the amino acid at position K47 corresponding to SEQ ID NO: 10 by best global alignment.

In a specific embodiment, extracellular domain of the envelope glycoprotein comprises an amino acid sequence as set forth in any one of SEQ ID NO: 2, SEQ ID NO: 3, SEQ ID NO: 11, SEQ ID NO: 12, SEQ ID NO: 17, SEQ ID NO: 18, SEQ ID NO: 19 and SEQ ID NO: 20.

In a specific embodiment, the extracellular domain of the envelope glycoprotein comprises an amino acid sequence as set forth in SEQ ID NO:11, or an amino acid sequence having at least 75%, 80%, 90%, 91%, 92%, 93%, 94%, 95%, 96%, 97%, 98% or 99% identity to the amino acid sequence as set forth in SEQ ID NO: 11; the amino acid sequence as set forth in SEQ ID NO: 11 is the amino acid sequence of SEQ ID NO: 1 after the following modifications: deletion of the amino acid at position K47, substitution from threonine (T) to asparagine (N) at position 214 (relative to the state before the K47 deletion) and substitution from threonine (T) to alanine (A) at position 352 (relative to the state before the K47 deletion).

In a specific embodiment, extracellular domain of the envelope glycoprotein comprises an amino acid sequence as set forth in SEQ ID NO:12, or an amino acid sequence having at least 75%, 80%, 90%, 91%, 92%, 93%, 94%, 95%, 96%, 97%, 98% or 99% identity to the amino acid sequence as set forth in SEQ ID NO:12; the amino acid sequence as set forth in SEQ ID NO: 12 is the amino acid sequence of SEQ ID NO: 1 after the following modifications: deletion of the amino acid at position K47, substitution from threonine (T) to asparagine (N) at position 214 (relative to the state before the K47 deletion), substitution from threonine (T) to alanine (A) at position 352 (relative to the state before the K47 deletion), substitution from lysine (K) to threonine (T) at position 50 (relative to the state before the K47 deletion) and substitution from serine (S) to threonine (T) at position 146 (relative to the state before the K47 deletion).

In a specific embodiment, extracellular domain of the envelope glycoprotein comprises an amino acid sequence as set forth in SEQ ID NO:2, or an amino acid sequence having at least 75%, 80%, 90%, 91%, 92%, 93%, 94%, 95%, 96%, 97%, 98% or 99% identity to the amino acid sequence as set forth in SEQ ID NO:2; the amino acid sequence as set forth in SEQ ID NO: 2 is the amino acid sequence of SEQ ID NO: 1 after the following modifications: substitution from arginine (R) to glutamine (Q) at position 354; substitution from threonine (T) to asparagine (N) at position 214 and substitution from threonine (T) to alanine (A) at position 352.

In a specific embodiment, extracellular domain of the envelope glycoprotein comprises an amino acid sequence as set forth in SEQ ID NO:3, or an amino acid sequence having at least 75%, 80%, 90%, 91%, 92%, 93%, 94%, 95%, 96%, 97%, 98% or 99% identity to the amino acid sequence as set forth in SEQ ID NO:3; the amino acid sequence as set forth in SEQ ID NO: 3 is the amino acid sequence of SEQ ID NO: 1 after the following modifications: substitution from arginine (R) to glutamine (Q) at position 354; substitution from threonine (T) to asparagine (N) at position 214; substitution from threonine (T) to alanine (A) at position 352; substituted from lysine (K) to threonine (T) at position 50 and substituted from serine (S) to threonine (T) at position 146.

In a specific embodiment, extracellular domain of the envelope glycoprotein comprises an amino acid sequence as set forth in SEQ ID NO:15, or an amino acid sequence having at least 75%, 80%, 90%, 91%, 92%, 93%, 94%, 95%, 96%, 97%, 98% or 99% identity to the amino acid sequence as set forth in SEQ ID NO:15; the amino acid sequence as set forth in SEQ ID NO: 15 is the amino acid sequence of SEQ ID NO: 1 after the following modifications: deletion of the amino acid at position R354; substitution from threonine (T) to asparagine (N) at position 214 (relative to the state before the R354 deletion) and substitution from threonine (T) to alanine (A) at position 352 (relative to the state before the R354 deletion).

In a specific embodiment, extracellular domain of the envelope glycoprotein comprises an amino acid sequence as set forth in SEQ ID NO:16, or an amino acid sequence having at least 75%, 80%, 90%, 91%, 92%, 93%, 94%, 95%, 96%, 97%, 98% or 99% identity to the amino acid sequence as set forth in SEQ ID NO: 16; the amino acid sequence as set forth in SEQ ID NO: 16 is the amino acid sequence of SEQ ID NO: 1 after the following modifications: deletion of the amino acid at position R354; substitution from threonine (T) to asparagine (N) at position 214 (relative to the state before the R354 deletion); substitution from threonine (T) to alanine (A) at position 352 (relative to the state before the R354 deletion); substituted from lysine (K) to threonine (T) at position 50 (relative to the state before the R354 deletion) and substituted from serine (S) to threonine (T) at position 146 (relative to the state before the R354 deletion).

In a specific embodiment, extracellular domain of the envelope glycoprotein comprises an amino acid sequence as set forth in SEQ ID NO:17, or an amino acid sequence having at least 75%, 80%, 90%, 91%, 92%, 93%, 94%, 95%, 96%, 97%, 98% or 99% identity to the amino acid sequence as set forth in SEQ ID NO: 17; the amino acid sequence as set forth in SEQ ID NO: 17 is the amino acid sequence of SEQ ID NO: 10 after the following modifications: deletion of the amino acid at position K47; substitution from lysine (K) to asparagine (N) at position 214 (relative to the state before the K47 deletion); substitution from threonine (T) to alanine (A) at position 352 (relative to the state before the K47 deletion); substituted from lysine (K) to threonine (T) at position 50 (relative to the state before the K47 deletion) and substituted from serine (S) to threonine (T) at position 146 (relative to the state before the K47 deletion).

In a specific embodiment, extracellular domain of the envelope glycoprotein comprises an amino acid sequence as set forth in SEQ ID NO:18, or an amino acid sequence having at least 75%, 80%, 90%, 91%, 92%, 93%, 94%, 95%, 96%, 97%, 98% or 99% identity to the amino acid sequence as set forth in SEQ ID NO:18; the amino acid sequence as set forth in SEQ ID NO: 18 is the amino acid sequence of SEQ ID NO: 10 after the following modifications: deletion of the amino acid at position K47; substitution from lysine (K) to asparagine (N) at position 214 (relative to the state before the K47 deletion) and substitution from threonine (T) to alanine (A) at position 352 (relative to the state before the K47 deletion).

In a specific embodiment, extracellular domain of the envelope glycoprotein comprises an amino acid sequence as set forth in SEQ ID NO:19, or an amino acid sequence having at least 75%, 80%, 90%, 91%, 92%, 93%, 94%, 95%, 96%, 97%, 98% or 99% identity to the amino acid sequence as set forth in SEQ ID NO:19; the amino acid sequence as set forth in SEQ ID NO: 19 is the amino acid sequence of SEQ ID NO: 10 after the following modifications: substitution from arginine (R) to glutamine (Q) at position 354; substitution from lysine (K) to asparagine (N) at position 214; substitution from threonine (T) to alanine (A) at position 352; substituted from lysine (K) to threonine (T) at position 50 and substituted from serine (S) to threonine (T) at position 146.

In a specific embodiment, extracellular domain of the envelope glycoprotein comprises an amino acid sequence as set forth in SEQ ID NO: 20, or an amino acid sequence having at least 75%, 80%, 90%, 91%, 92%, 93%, 94%, 95%, 96%, 97%, 98% or 99% identity to the amino acid sequence as set forth in SEQ ID NO: 20; the amino acid sequence as set forth in SEQ ID NO: 20 is the amino acid sequence of SEQ ID NO: 10 after the following modifications: substitution from arginine (R) to glutamine (Q) at position 354; substitution from lysine (K) to asparagine (N) at position 214 and substitution from threonine (T) to alanine (A) at position 352.

In a specific embodiment, extracellular domain of the envelope glycoprotein comprises an amino acid sequence as set forth in SEQ ID NO: 13, or an amino acid sequence having at least 75%, 80%, 90%, 91%, 92%, 93%, 94%, 95%, 96%, 97%, 98% or 99% identity to the amino acid sequence as set forth in SEQ ID NO: 13; the amino acid sequence as set forth in SEQ ID NO: 13 is the amino acid sequence of SEQ ID NO: 10 after the following modifications: deletion of the amino acid at position R354; substitution from lysine (K) to asparagine (N) at position 214 (relative to the state before the R354 deletion) and substitution from threonine (T) to alanine (A) at position 352 (relative to the state before the R354 deletion).

In a specific embodiment, extracellular domain of the envelope glycoprotein comprises an amino acid sequence as set forth in SEQ ID NO: 14, or an amino acid sequence having at least 75%, 80%, 90%, 91%, 92%, 93%, 94%, 95%, 96%, 97%, 98% or 99% identity to the amino acid sequence as set forth in SEQ ID NO: 14; the amino acid sequence as set forth in SEQ ID NO: 14 is the amino acid sequence of SEQ ID NO: 10 after the following modifications: deletion of the amino acid at position R354; substitution from lysine (K) to asparagine (N) at position 214 (relative to the state before the R354 deletion); substitution from threonine (T) to alanine (A) at position 352 (relative to the state before the R354 deletion); substituted from lysine (K) to threonine (T) at position 50 (relative to the state before the R354 deletion) and substituted from serine (S) to threonine (T) at position 146 (relative to the state before the R354 deletion).

In another aspect, the disclosure provides a nucleic acid molecule, encoding the envelope glycoprotein of the disclosure.

In another aspect, the disclosure provides a vector, comprising the nucleic acid molecule of the disclosure, or expressing the envelope glycoprotein of the disclosure.

In a specific embodiment, the vector is a lentiviral vector or a retroviral vector.

In another aspect, the disclosure provides a targeting vector, comprising: a first molecule that binds to an endocytic receptor of a target cell and a second molecule that is non complement inactivated and promotes the release of a substance carried by the targeting vector into the cytoplasm.

The second molecule promotes endosomal escape or lysosomal escape of the targeting vector, and prevents the targeting vector from being inactivated by complement. The second molecule is the envelope glycoprotein mutant of the disclosure, particularly the envelope glycoprotein mutant of vesicular stomatitis virus, which comprises first mutation that prevents the envelope glycoprotein from mediating complement inactivation.

The first mutation: the extracellular domain of the envelope glycoprotein comprises the amino acid sequence as set forth in SEQ ID NO: 1 or an amino acid sequence having at least 50% identity to the amino acid sequence as set forth in SEQ ID NO: 1, and the amino acid sequence comprises at least one mutated amino acid as following:
a) the amino acid at position 214 of SEQ ID NO: 1;
b) the amino acid located at position 214 corresponding to SEQ ID NO: 1 by best global alignment;
c) the amino acid at position 352 of SEQ ID NO: 1;
d) the amino acid located at position 352 corresponding to SEQ ID NO: 1 by best global alignment;
e) the amino acid at position 50 of SEQ ID NO: 1;
f) the amino acid located at position 50 corresponding to SEQ ID NO: 1 by best global alignment;
g) the amino acid at position 146 of SEQ ID NO: 1;
h) the amino acid located at position 146 corresponding to SEQ ID NO: 1 by best global alignment.

In a specific embodiment, the mutation is an insertion, deletion or substitution.

In a specific embodiment, the amino acid sequence comprises at least one substituted amino acid as following:
a) the amino acid at position 214 of SEQ ID NO: 1;
b) the amino acid located at position 214 corresponding to SEQ ID NO: 1 by best global alignment;
c) the amino acid at position 352 of SEQ ID NO: 1;
d) the amino acid located at position 352 corresponding to SEQ ID NO: 1 by best global alignment;
e) the amino acid at position 50 of SEQ ID NO: 1;
f) the amino acid located at position 50 corresponding to SEQ ID NO: 1 by best global alignment;
g) the amino acid at position 146 of SEQ ID NO: 1;
h) the amino acid located at position 146 corresponding to SEQ ID NO: 1 by best global alignment.

In a specific embodiment, the amino acid sequence comprises one or more mutated amino acid as following: substitution of the amino acid at position T214, substitution of the amino acid at position T352, substitution of the amino acid at position K50, substitution of the amino acid at position S146.

In a specific embodiment, the amino acid sequence comprises one or more mutated amino acid as following: the amino acid at position 214 is substituted from threonine (T) to asparagine (N), and/or the amino acid at position 352 is substituted from threonine (T) to alanine (A), and/or the amino acid at position 50 is substituted from lysine (K) to threonine (T), and/or the amino acid at position 146 is substituted from serine (S) to threonine (T).

In a specific embodiment, the amino acid sequence comprises one of the mutated amino acid combinations as following:
(1) the amino acid at position 214 is substituted from threonine (T) to asparagine (N) and the amino acid at position 352 is substituted from threonine (T) to alanine (A);
(2) the amino acid at position 214 is substituted from threonine (T) to asparagine (N), the amino acid at position 352 is substituted from threonine (T) to alanine (A), the amino acid at position 50 is substituted from lysine (K) to threonine (T) and the amino acid at position 146 is substituted from serine (S) to threonine (T).

In a specific embodiment, the amino acid sequence having at least 50% identity to the amino acid sequence as set forth in SEQ ID NO: 1, the extracellular domain of the envelope glycoprotein of Cocal virus comprises the amino acid sequence as set forth in SEQ ID NO: 10:

KFSIVFPQSQKGNWKNVPSSYHYCPSSSDQNWHNDLLGITMKVKMP
KTHKAIQADGWMCHAAKWITTCDFRWYGPKYITHSIHSIQPTSEQCKESIKQT
KQGTWMSPGFPPQNCGYATVTDSVAVVVQATPHHVLVDEYTGEWIDSQFPNG
KCETEECETVHNSTVWYSDYKVTGLCDATLVDTEITFFSEDGKKESIGKPNTG
YRSNYFAYEKGDKVCKMNYCKHAGVRLPSGVWFEFVDQDVYAAAKLPECP
VGATISAPTQTSVDVSLILDVERILDYSLCQETWSKIRSKQPVSPVDLSYLAPK
NPGTGPAFTIINGTLKYFETRYIRIDIDNPIISKMVGKISGSQTERELWTEWFPYE
GVEIGPNGILKTPTGYKFPLFMIGHGMLDSDLHKTSQAEVFEHPHLAEAPKQL

PEEETLFFGDTGISKNPVELIEGWFSSWKSTVVTFFFAIGVFILLYVVARIVIAVR
YRYQGSNNKRIYNDIEMSRFRK.

In a specific embodiment, SEQ ID NO: 10 comprises at least one mutated amino acid as following:
a) the amino acid at position 214 of SEQ ID NO: 10;
b) the amino acid located at position 214 corresponding to SEQ ID NO: 10 by best global alignment;
c) the amino acid at position 352 of SEQ ID NO: 10;
d) the amino acid located at position 352 corresponding to SEQ ID NO: 10 by best global alignment;
e) the amino acid at position 50 of SEQ ID NO: 10;
f) the amino acid located at position 50 corresponding to SEQ ID NO: 10 by best global alignment;
g) the amino acid at position 146 of SEQ ID NO: 10;
h) the amino acid located at position 146 corresponding to SEQ ID NO: 10 by best global alignment.

In a specific embodiment, the mutation is an insertion, deletion or substitution.

In a specific embodiment, SEQ ID NO: 10 comprises at least one substituted amino acid as following:
a) the amino acid at position 214 of SEQ ID NO: 10;
b) the amino acid located at position 214 corresponding to SEQ ID NO: 10 by best global alignment;
c) the amino acid at position 352 of SEQ ID NO: 10;
d) the amino acid located at position 352 corresponding to SEQ ID NO: 10 by best global alignment;
e) the amino acid at position 50 of SEQ ID NO: 10;
f) the amino acid located at position 50 corresponding to SEQ ID NO: 10 by best global alignment;
g) the amino acid at position 146 of SEQ ID NO: 10;
h) the amino acid located at position 146 corresponding to SEQ ID NO: 10 by best global alignment.

In a specific embodiment, SEQ ID NO: 10 comprises one or more mutated amino acid as following: substitution of the amino acid at position K214, substitution of the amino acid at position T352, substitution of the amino acid at position K50, substitution of the amino acid at position S146.

In a specific embodiment, SEQ ID NO: 10 comprises one or more mutated amino acid as following: the amino acid at position 214 is substituted from lysine (K) to asparagine (N), and/or the amino acid at position 352 is substituted from threonine (T) to alanine (A), and/or the amino acid at position 50 is substituted from lysine (K) to threonine (T), and/or the amino acid at position 146 is substituted from serine (S) to threonine (T).

In a specific embodiment, SEQ ID NO: 10 comprises one of the mutated amino acid combinations as following:
(1) substitution of the amino acid at position K214 and T352;
(2) substitution of the amino acid at position K214, T352, K50, and S146.

In a specific embodiment, SEQ ID NO: 10 comprises one of the mutated amino acid combinations as following:
the amino acid at position 214 is substituted from lysine (K) to asparagine (N) and the amino acid at position 352 is substituted from threonine (T) to alanine (A);
the amino acid at position 214 is substituted from lysine (K) to asparagine (N), the amino acid at position 352 is substituted from threonine (T) to alanine (A), the amino acid at position 50 is substituted from lysine (K) to threonine (T) and the amino acid at position 146 is substituted from serine (S) to threonine (T).

In a specific embodiment, the role of the second molecule is to promote endosomal escape or lysosomal escape of the targeting vector, and enhance the ability of the targeting vector to antagonize inactivation by complement. The second molecule is the envelope glycoprotein mutant of the disclosure, particularly the envelope glycoprotein mutant of vesicular stomatitis virus, which comprises any of the aforementioned first mutations that enhance the ability of the envelope glycoprotein to antagonize complement inactivation. The enhanced ability to antagonize complement inactivation is relative to before the occurrence of any of the aforementioned first mutations.

The envelope glycoprotein mutant of the disclosure also comprises the second mutation which weakens the ability of the envelope glycoprotein to be recognized by a receptor, such as a weakened or absent ability to bind LDL-R, allowing it to only retain the ability to escape endosomes or lysosomes, thereby relying entirely on the first molecule to exert targeted effects. By carrying the mutated envelope glycoprotein, the targeting ability of the vector is further enhanced, enabling it to infect only specific target cells. In a specific embodiment, the second mutation is mutated VSVG, this mutation disrupts the binding ability of VSVG to LDL-R, while not affecting the lysosomal escape ability of VSVG. The mutation is in the form of insertion, deletion or substitution.

The second mutation: the extracellular domain of the envelope glycoprotein comprises the amino acid sequence as set forth in SEQ ID NO: 1 or an amino acid sequence having at least 50% identity to the amino acid sequence as set forth in SEQ ID NO: 1, and the amino acid sequence comprises at least one mutated amino acid as following:
1) substitution/deletion of the amino acid at position H8, substitution/deletion of the amino acid at position N9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position S183, substitution/deletion of the amino acid at position S179, substitution/deletion of the amino acid at position N180, substitution/deletion of the amino acid at position I182, substitution/deletion of the amino acid at position M184, substitution/deletion of the amino acid at position Y209, substitution/deletion of the amino acid at position I347, substitution/deletion of the amino acid at position T350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 of SEQ ID NO: 1;
2) substitution/deletion of the amino acid at position H8, substitution/deletion of the amino acid at position N9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position S183, substitution/deletion of the amino acid at position S179, substitution/deletion of the amino acid at position N180, substitution/deletion of the amino acid at position I182, substitution/deletion of the amino acid at position M184, substitution/deletion of the amino acid at position Y209, substitution/deletion of the amino acid at position I347, substitution/deletion of the amino acid at position T350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 corresponding to SEQ ID NO: 1 by best global alignment.

In a specific embodiment, the amino acid sequence comprises at least one mutated amino acid as following:
1) substitution of the amino acid at position H8, substitution of the amino acid at position N9, substitution of the amino acid at position Q10, substitution of the amino acid at position K47, deletion of the amino acid at position K47, substitution of the amino acid at position K50, substitution of the amino acid at position A51, substitution of the amino acid at position S183; substitution of the amino acid at position S179; substitution of the amino acid at position N180, substitution of the amino acid at position I182, substitution of the amino acid at position M184, substitution of the amino acid at position Y209, substitution of the amino acid at position I347, substitution of the amino acid at position T350, substitution of the amino acid at position T352, substitution of the amino acid at position E353, substitution of the amino acid at position R354, deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 of SEQ ID NO: 1;
2) substitution of the amino acid at position H8, substitution of the amino acid at position N9, substitution of the amino acid at position Q10, substitution of the amino acid at position K47, deletion of the amino acid at position K47, substitution of the amino acid at position K50, substitution of the amino acid at position A51, substitution of the amino acid at position S183; substitution of the amino acid at position S179; substitution of the amino acid at position N180, substitution of the amino acid at position I182, substitution of the amino acid at position M184, substitution of the amino acid at position Y209, substitution of the amino acid at position I347, substitution of the amino acid at position T350, substitution of the amino acid at position T352, substitution of the amino acid at position E353, substitution of the amino acid at position R354, deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 corresponding to SEQ ID NO: 1 by best global alignment.

In a specific embodiment, the amino acid sequence comprises one or more mutated amino acid as following: substitution/deletion of the amino acid at position K47, substitution/deletion of the amino acid at position R354.

In a specific embodiment, the amino acid sequence comprises one or more mutated amino acid as following: the amino acid at position 47 is substituted from lysine (K) to glutamine (Q); the amino acid at position 354 is substituted from arginine (R) to glutamine (Q), deletion of the amino acid at position 47, deletion of the amino acid at position 354.

In a specific embodiment, the amino acid sequence comprises one of the mutated amino acid as following:
(1) substitution of the amino acid at position K47;
(2) substitution of the amino acid at position R354.

In a specific embodiment, the amino acid sequence comprises one of the mutated amino acid as following:
(1) the amino acid at position 47 is substituted from lysine (K) to glutamine (Q);
(2) the amino acid at position 354 is substituted from arginine (R) to glutamine (Q).

In a specific embodiment, the amino acid sequence comprises at least one mutated amino acid as following:
deletion of amino acids at position 331-364, deletion of amino acids at position 344-354, substitution of the amino acid at position K47, deletion of the amino acid at position K47, substitution of the amino acid at position R354, deletion of the amino acid at position R354 of SEQ ID NO: 1;
deletion of amino acids at position 331-364, deletion of amino acids at position 344-354, substitution of the amino acid at position K47, deletion of the amino acid at position K47, substitution of the amino acid at position R354, deletion of the amino acid at position R354 corresponding to SEQ ID NO: 1 by best global alignment.

In a specific embodiment, the amino acid sequence comprises at least one mutated amino acid as following:
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 of SEQ ID NO: 1; and
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 corresponding to SEQ ID NO: 1 by best global alignment.

In a specific embodiment, the amino acid sequence comprises one of the mutated amino acid as following: (1) deletion of the amino acid at position K47 of SEQ ID NO: 1;
deletion of the amino acid at position K47 corresponding to SEQ ID NO: 1 by best global alignment.

In a specific embodiment, the second mutation is an amino acid sequence having at least 50% identity to the amino acid sequence as set forth in SEQ ID NO: 1, SEQ ID NO: 10 comprises at least one mutated amino acid as following:
substitution/deletion of the amino acid at position Q8, substitution/deletion of the amino acid at position S9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position D183, substitution/deletion of the amino acid at position A179, substitution/deletion of the amino acid at position T180, substitution/deletion of the amino acid at position V182, substitution/deletion of the amino acid at position T184, substitution/deletion of the amino acid at position Y209, substitution/deletion of the amino acid at position I347, substitution/deletion of the amino acid at position S350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 of SEQ ID NO: 10;
substitution/deletion of the amino acid at position Q8, substitution/deletion of the amino acid at position S9, substitution/deletion of the amino acid at position Q10, substitution/deletion of the amino acid at position K47, substitution/deletion of the amino acid at position K50, substitution/deletion of the amino acid at position A51, substitution/deletion of the amino acid at position D183, substitution/deletion of the amino acid at position A179, substitution/deletion of the amino acid at position T180, substitution/deletion of the amino acid at position V182, substitution/deletion of the amino acid at position T184, substitution/deletion of the amino acid at position Y209, substitution/deletion of the amino acid at position I347, substitution/deletion of the amino acid at position S350, substitution/deletion of the amino acid at position T352, substitution/deletion of the amino acid at position E353, substitution/deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids at position 345-353 corresponding to SEQ ID NO: 10 by best global alignment.

In a specific embodiment, SEQ ID NO: 10 comprises at least one mutated amino acid as following:
substitution of the amino acid at position Q8, substitution of the amino acid at position S9, substitution of the amino acid at position Q10, substitution or deletion of the amino acid at position K47, substitution of the amino acid at position K50, substitution of the amino acid at position A51, substitution of the amino acid at position D183, substitution of the amino acid at position A179, substitution of the amino acid at position T180, substitution of the amino acid at position V182, substitution of the amino acid at position T184, substitution of the amino acid at position Y209, substitution of the amino acid at position I347, substitution of the amino acid at position S350, substitution of the amino acid at position T352, substitution of the amino acid at position E353, substitution or deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids 345-353 of SEQ ID NO: 10;
substitution of the amino acid at position Q8, substitution of the amino acid at position S9, substitution of the amino acid at position Q10, substitution or deletion of the amino acid at position K47, substitution of the amino acid at position K50, substitution of the amino acid at position A51, substitution of the amino acid at position D183, substitution of the amino acid at position A179, substitution of the amino acid at position T180, substitution of the amino acid at position V182, substitution of the amino acid at position T184, substitution of the amino acid at position Y209, substitution of the amino acid at position I347, substitution of the amino acid at position S350, substitution of the amino acid at position T352, substitution of the amino acid at position E353, substitution or deletion of the amino acid at position R354, deletion of amino acids at position 1-18, deletion of amino acids at position 19-36, deletion of amino acids at position 37-51, deletion of amino acids at position 314-384, deletion of amino acids at position 321-374, deletion of amino acids at position 331-364, deletion of amino acids at position 344-354, deletion of amino acids 345-353 corresponding to SEQ ID NO: 10 by best global alignment.

In a specific embodiment, SEQ ID NO: 10 comprises at least one mutated amino acid as following:
deletion of amino acids at position 331-364, deletion of amino acids at position 344-354, substitution of the amino acid at position K47, deletion of the amino acid at position K47, substitution of the amino acid at position R354, deletion of the amino acid at position R354 of SEQ ID NO:10;
deletion of amino acids at position 331-364, deletion of amino acids at position 344-354, substitution of the amino acid at position K47, deletion of the amino acid at position K47, substitution of the amino acid at position R354, deletion of the amino acid at position R354 corresponding to SEQ ID NO: 10 by best global alignment.

In a specific embodiment, SEQ ID NO: 10 comprises at least one mutated amino acid as following:
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 of SEQ ID NO:10; and
deletion of amino acids at position 331-364, deletion of amino acids at position 344-354, the amino acid at position 47 is substituted from lysine (K) to glutamine (Q), the amino acid at position 354 is substituted from arginine (R) to glutamine (Q), deletion of the amino acid at position R354, deletion of the amino acid at position K47 corresponding to SEQ ID NO: 10 by best global alignment.

In a specific embodiment, SEQ ID NO: 10 comprises one of the mutated amino acid as following:
deletion of the amino acid at position K47 of SEQ ID NO:10;
deletion of the amino acid at position K47 corresponding to SEQ ID NO: 10 by best global alignment.

In a specific embodiment, extracellular domain of the envelope glycoprotein (the second molecule) with a first mutation and a second mutation comprises an amino acid sequence as set forth in any one of SEQ ID NO: 2, SEQ ID NO: 3, SEQ ID NO: 11, SEQ ID NO: 12, SEQ ID NO: 17, SEQ ID NO: 18, SEQ ID NO: 19 and SEQ ID NO: 20.

In a specific embodiment, the weakens the ability of the envelope glycoprotein to be recognized by the receptor relative to before the occurrence of the second mutation.

The targeting vector is an enveloped virus, such as a retroviral vector or a lentiviral vector, preferably is a VSV vector; The second molecule is the envelope glycoprotein of virus, preferably the envelope glycoprotein of vesicular stomatitis virus (VSVG) and its mutations; the first molecule is not part of a viral envelope glycoprotein.

The envelope glycoprotein of virus, such as VSVG, can promote the fusion of the viral envelope with the endosome/lysosome membrane in endosomes/lysosomes, thereby facilitating the release of substances carried by the vector.

The mutation refers to a mutant that has at least 75% identity with the amino acid sequence of the non-mutant (wild-type). "At least 75% identity" means being 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identical to the amino acid sequence of the non-mutant (wild-type).

Since the receptor for VSVG, LDL-R, is widely expressed in various cells such as activated T cells, hepatocytes, cardiomyocytes, endothelial cells, and stem cells, as well as in many types of tumor cells, pseudotyped lentiviruses carrying VSVG and its mutations can infect multiple types of cells. Although VSVG pseudotyped viruses have broad-spectrum infectivity, many cells either do not express or have low expression of LDL-R, the receptor for VSVG, such as NK cells and resting T cells, making it difficult for VSVG pseudotyped viruses to infect them. In addition, due to the wide expression of LDL-R in various cells, VSVG pseudotyped viruses lack sufficient targeting ability. As an illustration, since multiple cell types express LDL-R, lentiviruses cannot accurately transfect only one specific type among them.

The role of the second molecule includes promoting endosomal escape or lysosomal escape of the targeting vector after it is endocytosed by the target cell, thereby enabling it to enter the cytoplasm. If a targeting vector, after being endocytosed, fails to undergo endosomal escape or lysosomal escape, it will eventually be degraded by lysosomes. Therefore, for effectively delivering the substances carried by the vector enter the cytoplasm, the vector needs to possess the ability of endosomal escape or lysosomal escape.

The disclosure utilizes the endosomal/lysosomal escape capability of lentiviruses, and modifies them by conjugating the first molecule to the lentiviruses to prepare targeting vector. The targeting vector can be flexibly applied to different scenarios involving various cells. By designing the first molecule according to the cells to be targeted, the scope of application is greatly expanded, and the targeting accuracy is also improved.

Endocytic receptors refer to membrane proteins expressed on the cell surface that can induce endocytosis (also known as pinocytosis) upon binding to antibodies, ligands, or specific substances, such as CD7, CD5, HER2, Mesothelin, etc.

Typically, endocytic receptors contain domains like YXXPhi, [D/E]XXXL[L/I], FXNPXY, etc. In addition, some membrane proteins, such as CD8 molecules, do not possess endocytic capability due to the lack of endocytic receptor domains. Therefore, in the screening of endocytic domains, CD8 molecules with random sequences chimerized at the end of their intracellular sequences are commonly used for screening.

Different cells express different and specific endocytic receptors. The first molecule can be designed based on the endocytic receptors of the target cells. Through antibodies or ligands (as part of the first molecule) that specifically bind to the endocytic receptors, the vector is endocytosed by the target cells, thereby enabling the vector to have infectivity to specific cells while having no infectivity to other cells.

Binding of the first molecule to the endocytic receptor triggers receptor-mediated endocytosis. Receptor-mediated endocytosis is a process by which cells specifically take up extracellular proteins or other compounds relying on receptors on the cell surface. The receptors on the cell surface are highly specific and form complexes upon binding to their corresponding ligands. Subsequently, this part of the plasma membrane invaginates to form coated pits, which then detach from the plasma membrane to form coated vesicles, taking extracellular substances into the cell. After entering the cell, the coated vesicles shed their coats and fuse with small vesicles of endosomes to form large endosomes or endosomes.

In a specific embodiment, the first molecule comprises a transmembrane peptide segment, an antibody or ligand that binds to endocytic receptor of target cell. In some embodiment, the first molecule further comprises extracellular hinge region. Proteins expressed on the membrane generally require a hinge regions, which facilitates the extension of membrane proteins. The CD8 hinge region is commonly used.

Without affecting its function, the amino acid sequence of the first molecule is not limited. As an illustration, when the first molecule comprises CD33 antibody, it can be a protein that has at least about 75%, 80%, 85%, 90%, 91%, 92%, 93%, 94%, 95%, 96%, 97%, 98%, 99%, or 99.5% identity with the amino acid sequence of the CD33 antibody.

Different target cells have different endocytic receptors. Common endocytic receptors such as: HER2, CD20, CD19, CD79A, CD79B, CD56, CD22, CD138, CD37, CD98, CD309, CD33, CD163, CD163B, CD5, CD7, CD169, CD204, CD205, CD209, CD280, CD302, TROP-2, CD19, NECTIN4, 5T4, CD30, TROP2, FR [alpha], STEAP1, ENPP3, GCC, SLC44A4, NaPi2b, CA9, SC-16, CD142, P-Cadherin, PSMA, ED-B, endothelin ETB, TN-C, Collagen IV, Periostin, CEACAM, c-MET, TDGF1, IGF1R, Mesothelin, TIM1, NCAM1, ZIP6, CD166, GPNMB, SDC1, glycosphingolipid, TfR, Ganglioside, CD74, The following are the results of the analysis: CLDN18, DPEP3, SLITRK6, PRL-R, LY75, CD48, MUC1, CDKs, B7-H4, STING, KAAG1, CD70, CDH3, LRRC15, EGFR or ASGPR.

In a specific embodiment, the endocytic receptor is CD7.

In a specific embodiment, the antibody is anti-CD7 single-domain antibody; the HCDR1 region of the anti-CD7 single-domain antibody comprises an amino acid sequence as set forth in SEQ ID NO:22, or an amino acid sequence having at least 75%, 80%, 90%, 91%, 92%, 93%, 94%, 95%, 96%, 97%, 98% or 99% identity to the amino acid sequence as set forth in SEQ ID NO:22; the HCDR2 region of the anti-CD7 single-domain antibody comprises an amino acid sequence as set forth in SEQ ID NO:23, or an amino acid sequence having at least 75%, 80%, 90%, 91%, 92%, 93%, 94%, 95%, 96%, 97%, 98% or 99% identity to the amino acid sequence as set forth in SEQ ID NO:23; the HCDR3 region of the anti-CD7 single-domain antibody comprises an amino acid sequence as set forth in SEQ ID NO:24, or an amino acid sequence having at least 75%, 80%, 90%, 91%, 92%, 93%, 94%, 95%, 96%, 97%, 98% or 99% identity to the amino acid sequence as set forth in SEQ ID NO:24.

In a specific embodiment, the anti-CD7 single-domain antibody (VHH) comprises an amino acid sequence as set forth in SEQ ID NO:21, or an amino acid sequence having at least 75%, 80%, 90%, 91%, 92%, 93%, 94%, 95%, 96%, 97%, 98% or 99% identity to the amino acid sequence as set forth in SEQ ID NO:21.

In a specific embodiment, the targeting is a lentiviral vector; first molecule expressed by the lentiviral vector is the transmembrane protein, and the second molecule expressed by the lentiviral vector is a viral envelope glycoprotein, which has the ability to promote the endosomal escape or lysosomal escape of targeting vectors. When the target cells do not express the receptor of the viral envelope glycoprotein, the targeting vector can achieve viral infection of the target cells through endocytosis mediated by the first molecule.

In a specific embodiment, the transmembrane protein is selected from CD7 antibody, CD79B antibody, CD33 antibody, ASGRP antibody, ASGRP ligand, Mesothelin antibody, HER2 antibody; the transmembrane protein can also be a protein having at least about 75%, 80%, 85%, 90%, 91%, 92%, 93%, 94%, 95%, 96%, 97%, 98%, 99%, or 99.5% identity to the amino acid sequence of at least one of the CD8 signal peptide of the CD7 antibody, the TH-69 heavy chain (VH, SEQ ID NO: 5), the GS linker, the TH-69 light chain (VL, SEQ ID NO: 7), the CD8 hinge region, and the CD8 transmembrane region; the transmembrane protein can also be a protein having at least about 75%, 80%, 85%, 90%, 91%, 92%, 93%, 94%, 95%, 96%, 97%, 98%, 99%, or 99.5% identity to the amino acid sequence of at least one of the CD8 signal peptide of the CD33 antibody, Gemtuzumab light chain (VL), GS linker, Gemtuzumab heavy chain (VH), CD8 hinge region, and CD8 transmembrane region; the transmembrane protein can also be a protein having at least about 75%, 80%, 85%, 90%, 91%, 92%, 93%, 94%, 95%, 96%, 97%, 98%, 99%, or 99.5% identity to the amino acid sequence of at least one of the CD8 signal peptide of the CD79B antibody, SN-8 heavy chain (VH), GS linker, SN-8 light chain (VL), CD8 hinge region, and CD8 transmembrane region; the transmembrane protein can also be a protein having at least about 75%, 80%, 85%, 90%, 91%, 92%, 93%, 94%, 95%, 96%, 97%, 98%, 99%, or 99.5% identity to the amino acid sequence of at least one of the CD8 signal peptide of the ASGRP antibody, ASGPR light chain B11 (VL), GS linker, ASGPR heavy chain (VH), CD8 hinge region, and CD8 transmembrane region. the transmembrane protein can also be a protein having at least about 75%, 80%, 85%, 90%, 91%, 92%, 93%, 94%, 95%, 96%, 97%, 98%, 99%, or 99.5% identity to the amino acid sequence of at least one of the CD8 signal peptide of the HER2 antibody, Pertuzumab light chain (VL), GS linker, Pertuzumab heavy chain (VH), CD8 hinge region, and CD8 transmembrane region; the transmembrane protein can also be a protein having at least about 75%, 80%, 85%, 90%, 91%, 92%, 93%, 94%, 95%, 96%, 97%, 98%, 99%, or 99.5% identity to the amino acid sequence of at least one of the CD8 signal peptide of the MESOTHELI antibody, PE38 heavy chain (VH), GS linker, PE38 light chain (VL), CD8 hinge region, and CD8 transmembrane region.

Taking lentiviral infection of T cells as an example, the lentiviral envelope expresses mutated VSVG and CD7 antibody. When the CD7 antibody binds to the CD7 antigen on the surface of T cells, it can mediate the endocytosis of the lentivirus. Then, the mutated VSVG mediates lysosomal escape, thereby completing the targeted infection of T cells by the lentivirus. Moreover, the lentivirus only infects T cells expressing CD7 and has no infectivity to cells that do not express CD7. Meanwhile, the mutated VSVG does not cause the lentivirus to be inactivated by complement in the serum.

The substances carried by the targeting vector are not limited and can be selected according to actual needs, such as small molecule compounds, proteins, polypeptides, RNA or DNA, including chimeric antigen receptors (CAR) and T cell receptors (TCR), etc. In a specific embodiment, the substance comprises the polynucleotide encoding the chimeric antigen receptor. The type of CAR is not limited, and the CAR comprises an antigen-binding domain. In some embodiments, the antigen-binding domain is a single-chain fragment variable (scFv) containing the variable regions of the heavy and light chains that specifically bind to the desired antigen. The scFv is selected from monoclonal antibodies, chimeric monoclonal antibodies, humanized monoclonal antibodies, human antibodies, nanobodies, and synthetic antibodies. In some embodiments, the CAR further comprises the transmembrane domain (e.g., CD8 transmembrane domain) and the signaling domain, wherein the signaling domains are based on immunoreceptor tyrosine-based activation motifs (ITAMs) (e.g., CD3ζ). In some embodiments, the CAR comprises one or more co-stimulatory domains. The CAR is not limited by the type of co-stimulatory domain. In fact, any co-stimulatory domain known in the art can be used, including, but not limited to CD28, 4-1BB, DAP10, DAP12. In some embodiments, the CAR can be first, second, third, or fourth generation. In some embodiments, the CAR can be monospecific, bispecific, or multispecific.

The small molecule compound refers to an organic compound molecule with a molecular weight of less than 900 Daltons, especially a small molecule that can be used as a drug.

In a specific embodiment, the target cells are lymphocytes, myeloid cells, hematopoietic stem/progenitor cells or non-blood cells, especially myeloid cells, hematopoietic stem/progenitor cells or non blood cells, including normal cells and tumor cells.

In another aspect, the disclosure provides a preparation method of the targeting vector, comprising the following steps:
designing the first molecule and the second molecule according to the endocytic receptor of the target cell,
assembling the first molecule and the second molecule with the substances carried by the vector to prepare the targeting vector.

The designing of the first molecule and second molecule comprises mutating their amino acid sequences. By designing the first molecule, which is to modify antibodies/ligands capable of targeting and recognizing specific receptors on the viral surface, and designing a mutant viral envelope glycoprotein with restricted receptor binding but possessing the ability to escape endosomes/lysosomes and complement inactivation as the second molecule, viruses can achieve targeted infection of target cells relying on the first molecule, regardless of whether the target cells express the receptor for the envelope glycoprotein carried by the virus. Meanwhile, the virus will not infect other cells, which greatly improves targeting precision, and it will not be inactivated by complement in the serum.

In a specific embodiment, the mutation includes any one of the aforementioned first mutation and second mutation.

The assembly of enveloped vectors, taking VSVG as an example: the targeting expression vector can be constructed by mixing the plasmid expressing antibodies/ligands that bind to endosomal receptor with the lentiviral packaging plasmids, such as psPAX2 and pMD.2G (VSVG or VSVG mutants), and the lentiviral expression vector.

Enveloped vectors, such as VSVG pseudotyped lentiviral vectors, enter cells through clathrin-mediated endocytosis via the binding of VSVG to LDL-R on the cell membrane surface. After acidification of the endosome formed by endocytosis, the conformation of VSVG changes, leading to the fusion of the viral envelope with the endosomal membrane, thereby enabling the virus to escape from the endosome/lysosome and enter the cell nucleus through the nuclear pore.

In another aspect, the targeting vector can be used for drug delivery or vaccine delivery, especially for delivering small molecule compounds, proteins, polypeptides, RNA or DNA.

In another aspect, the disclosure provides a method of introducing a substance into a cell, the method comprising: contacting the cell with a targeting vector.

In a specific embodiment, the cells are mammalian cells.

In a specific embodiment, the cells are normal cells or cancer cells.

In a specific embodiment, the cell are T cells, NK cells, B cells, macrophages, granulocytes, dendritic cells, hematopoietic stem cells, hepatocytes, islet cells, neurons or muscle cells.

In a specific embodiment, the contact is performed in vivo or in vitro. As an illustration, the targeting vector can be administered intravenously, intraperitoneally, intratumorally, intraosseously, or intranodally, enabling it to enter the body to make contact with the target cells; or the target virus can directly infect the target cells in vitro.

The term "mammal" refers to any mammalian species such as humans, mice, rats, dogs, cats, hamsters, guinea pigs, rabbits, domestic animals, etc.

In another aspect, the disclosure provides a host cell, comprising or expressing the envelope glycoprotein of the disclosure, or comprising the nucleic acid molecule of the disclosure, or comprising the targeting vector of the disclosure.

In another aspect, the disclosure provides a composition, comprising the envelope glycoprotein, the nucleic acid molecule or the host cell of the disclosure, or any combination thereof. The composition can be used as a medicament. The composition can be used for preparing medicaments for gene therapy, immunotherapy, cell therapy, treatment of genetic deficiency diseases, or treatment of cancer.

In another aspect, the disclosure provides a method for treating a disease suffered by a subject or killing disease cells in a subject, the method comprises administering the composition of the disclosure.

In a specific embodiment, the disease comprises cancer, and the cancer comprises hematological cancer and solid cancer.

In a specific embodiment, the administration is selected from at least one of oral, nasal, intravenous, intraperitoneal, intracerebral (intraparenchymal), intraventricular, intramuscular, intraocular, intraarterial, portal vein, intralesional, administration via a sustained release system, and administration via an implantable device.

The term "patient" refers to a subject who is suffering from a disease, disorder, or condition, is at risk of developing a disease, disorder, or condition, or otherwise in need of the composition or therapeutic method of the present invention; "patient" includes, but is not limited to, mammals such as humans or non-human mammals (e.g., domestic animals, agricultural animals, or wild animals), as well as birds and aquatic animals.

Throughout this specification, references to "some specific aspects," "one specific aspect," "embodiment," "specific embodiment," "related embodiment," "a certain embodiment," "another embodiment," "other embodiments," or combinations thereof mean that the specific feature, structure, or characteristic described in connection with the embodiment is included in at least one embodiment of the present invention. Therefore, the appearances of the aforementioned phrases in various places throughout this specification are not necessarily all referring to the same embodiment. Furthermore, the specific features, structures, or characteristics may be combined in any suitable manner in one or more embodiments.

This scheme performs reasonable mutations on the envelope glycoprotein of the virus and assembles it into a targeting vector, which has the following beneficial effects:
1. Effectively enhance the resistance of the targeting vector to complement-mediated inactivation, and improve its infection efficiency in serum;
2. Promote efficient in vivo targeted delivery.

### BRIEF DESCRIPTION OF THE DRAWINGS

FIG.1 is a schematic diagram of a targeting vector with an envelope containing an anti-CD7 antibody.
FIG.2 is the detection results of the infection efficiency of the VSVG mutant 1 lentivirus, VSVG mutant 3 lentivirus and VSVG mutant 4 lentivirus described in Example 1 in infecting CD7+Jurkat cells in the presence of serum.
FIG.3 is the detection results of the infection efficiency of the VSVG mutant 5 lentivirus and VSVG mutant 6 lentivirus described in Example 2 in infecting CD7+Jurkat cells in the presence of serum.

### DETAILED DESCRIPTION

A lentivirus targeting CD7+ cells was constructed using a VSVG mutant, wherein the VSVG mutant lost receptor recognition ability and simultaneously carried mutations capable of antagonizing complement-mediated inactivation, thereby enabling the vector to efficiently target and infect the target cells in the presence of serum.

### 1. Design a membrane-expressed anti-CD7 single-domain antibody

The membrane-expressed anti-CD7 single-domain antibody comprises, from the N-terminus to the C-terminus, a CD8 signal peptide, an anti-CD7 single-domain antibody (VHH), a CD8 hinge region, and a CD8 transmembrane region.
wherein the amino acid sequence of the CD8 signal peptide is set forth in SEQ ID NO: 4:
MALPVTALLLPLALLLHAARP.

The HCDR1 region of said anti-CD7 single-domain antibody comprises an amino acid sequence as set forth in SEQ ID NO: 22, or an amino acid sequence having at least 75%, 80%, 90%, 91%, 92%, 93%, 94%, 95%, 96%, 97%, 98% or 99% identity to the amino acid sequence as set forth in SEQ ID NO: 22;
SEQ ID NO: 22: GRAFSVYAMA.

The HCDR2 region of said anti-CD7 single-domain antibody comprises an amino acid sequence as set forth in SEQ ID NO: 23, or an amino acid sequence having at least 75%, 80%, 90%, 91%, 92%, 93%, 94%, 95%, 96%, 97%, 98% or 99% identity to the amino acid sequence as set forth in SEQ ID NO: 23;
SEQ ID NO: 23: SIAGSSSTYYTN.

The HCDR3 region of said anti-CD7 single-domain antibody comprises an amino acid sequence as set forth in SEQ ID NO: 24, or an amino acid sequence having at least 75%, 80%, 90%, 91%, 92%, 93%, 94%, 95%, 96%, 97%, 98% or 99% identity to the amino acid sequence as set forth in SEQ ID NO: 24;
SEQ ID NO:24: SPRSNNGRETRHYDY.

The anti-CD7 single-domain antibody (VHH) comprises an amino acid sequence as set forth in SEQ ID NO: 21, or an amino acid sequence having at least 75%, 80%, 90%, 91%, 92%, 93%, 94%, 95%, 96%, 97%, 98% or 99% identity to the amino acid sequence as set forth in SEQ ID NO: 21;
SEQ ID NO:21 :

DVQLQESGGGLVQAGGSLRLSCAASGRAFSVYAMAWFRQAPGKER
EFVSSIAGSSSTYYTNYADSVKGRFTISRDNAKNTMYLQMDELKDEDTAVYY
CAASPRSNNGRETRHYDYWGQGTQVTVSS.

The amino acid sequence of the CD8 hinge region as set forth in SEQ ID NO: 8:
TTTPAPRPPTPAPTIASQPLSLRPEACRPAAGGAVHTRGLDFACD.

The amino acid sequence of the CD8 transmembrane region as set forth in SEQ ID NO: 9:
IYIWAPLAGTCGVLLLSLVITLYC.

### 2. Construct mutant VSVG

Construct VSVG mutant 1, VSVG mutant 2, VSVG mutant 3, VSVG mutant 4, VSVG mutant 5, VSVG mutant 6 and the control VSVG mutant 1.

The extracellular domain of the envelope glycoprotein of the wild-type vesicular stomatitis virus Indiana strain (Indiana-VSVG) comprises the amino acid sequence as set forth in SEQ ID NO: 1.

The full-length protein of the envelope glycoprotein of the wild-type vesicular stomatitis virus Indiana strain comprises the amino acid sequence as set forth in SEQ ID NO: 25.

Control VSVG mutant 1 (CTR-VSVG1): the extracellular domain of the envelope glycoprotein of the CTR-VSVG1 comprises the amino acid sequence as set forth in SEQ ID NO: 26. The amino acid sequence as set forth in SEQ ID NO: 26 is the amino acid sequence of SEQ ID NO: 1 with the amino acid at position 354 substituted from arginine (R) to glutamine (Q); that is, compared with the wild-type Indiana-VSVG, the extracellular domain of CTR-VSVG1 has an R354Q mutation.

VSVG mutant 1: the extracellular domain of the envelope glycoprotein of the VSVG mutant 1 comprises the amino acid sequence as set forth in SEQ ID NO: 2. The amino acid sequence as set forth in SEQ ID NO: 2 is the amino acid sequence of SEQ ID NO: 1 with the amino acid at position 354 substituted from arginine (R) to glutamine (Q), the amino acid at position 214 substituted from threonine (T) to asparagine (N) and the amino acid at position 352 substituted from threonine (T) to alanine (A); that is, compared with the wild-type Indiana-VSVG, the extracellular domain of VSVG mutant 1 has T214N, T352A and R354Q mutations.

VSVG mutant 2: the extracellular domain of the envelope glycoprotein of the VSVG mutant 2 comprises the amino acid sequence as set forth in SEQ ID NO: 3. The amino acid sequence as set forth in SEQ ID NO: 3 is the amino acid sequence of SEQ ID NO: 1 with the amino acid at position 354 substituted from arginine (R) to glutamine (Q), the amino acid at position 214 substituted from threonine (T) to asparagine (N), the amino acid at position 352 substituted from threonine (T) to alanine (A), the amino acid at position 50 substituted from lysine (K) to threonine (T) and the amino acid at position 146 substituted from serine (S) to threonine (T); that is, compared with the wild-type Indiana-VSVG, the extracellular domain of VSVG mutant 2 has K50T, S146T, T214N, T352A and R354Q mutations.

VSVG mutant 3: the extracellular domain of the envelope glycoprotein of the VSVG mutant 3 comprises the amino acid sequence as set forth in SEQ ID NO: 11. The amino acid sequence as set forth in SEQ ID NO: 11 is the amino acid sequence of SEQ ID NO: 1 with the lysine (K) at position 47 deleted, the amino acid at position 214 (relative to the state before the K47 deletion) substituted from threonine (T) to asparagine (N) and the amino acid at position 352 (relative to the state before the K47 deletion) substituted from threonine (T) to alanine (A); that is, compared with the wild-type Indiana-VSVG, the extracellular domain of VSVG mutant 3 has K47 deletion, T214N mutations and T352A mutations.

VSVG mutant 4: the extracellular domain of the envelope glycoprotein of the VSVG mutant 4 comprises the amino acid sequence as set forth in SEQ ID NO: 12. The amino acid sequence as set forth in SEQ ID NO: 12 is the amino acid sequence of SEQ ID NO: 1 with he lysine (K) at position 47 deleted, the amino acid at position 214 (relative to the state before the K47 deletion) substituted from threonine (T) to asparagine (N), the amino acid at position 352 (relative to the state before the K47 deletion) substituted from threonine (T) to alanine (A), the amino acid at position 50 (relative to the state before the K47 deletion) substituted from lysine (K) to threonine (T) and the amino acid at position 146 (relative to the state before the K47 deletion) substituted from serine (S) to threonine (T); that is, compared with the wild-type Indiana-VSVG, the extracellular domain of VSVG mutant 4 has K47 deletion, K50T mutations, S146T mutations, T214N mutations and T352A mutations.

### 3. Lentivirus packaging

A. the following four types of plasmids were prepared to package control VSVG lentivirus 1 and VSVG mutant 1 lentivirus, VSVG mutant 2 lentivirus, VSVG mutant 3 lentivirus and VSVG mutant 4 lentivirus:
   A mutant envelope plasmid containing a polynucleotide encoding the mutant VSVG and a polynucleotide encoding the membrane-expressed anti-CD7 single-domain antibody, pMDLg/pRRE packaging plasmid, pRSV-REV packaging plasmid, and the target plasmid pGClenti-GFP (GFP fluorescent protein) were carried; the mutant VSVG envelope plasmid was synthesized by conventional molecular cloning methods; the mutant VSVG includes the control VSVG mutant 1, VSVG mutants 1, VSVG mutants 2, VSVG mutants 3 or VSVG mutants 4.
B. control VSVG lentivirus 1 and VSVG mutant 1 lentivirus, VSVG mutant 2 lentivirus VSVG mutant 3 lentivirus and VSVG mutant 4 lentivirus were packaged:
   the four types of plasmids were mixed, and transfected them into the packaging cell line HEK-293T using PEI reagent;

The specific steps were as follows:
Day 0, 9 µg of the target plasmid, 4 µg of pMDLg/pRRE packaging plasmid, 2 µg of pRSV-REV packaging plasmid, and 2 µg of the mutant VSVG envelope plasmid were added into 1 mL of Opti-MEM medium (Opti-MEM alpha reduced-serum medium, GIBCO, catalog number: #SP0272). After shaking well, 64 µL of PEI reagent was added, pipetted to mix evenly, and stand for 10 minutes, then added to the culture medium of HEK-293T cells. The medium was replaced after 6 hours. The culture supernatant was collected 48 hours after transfection, filtered with a 0.45 µm filter membrane, centrifuged at 50,000 g for 2.5 hours, and discarded the supernatant;
the control VSVG lentivirus 1 whose viral envelope contains the control VSVG mutant 1, the VSVG mutant 1 lentivirus whose viral envelope contains VSVG mutant 1, the VSVG mutant 2 lentivirus whose viral envelope contains VSVG mutant 2, the VSVG mutant 3 lentivirus whose viral envelope contains VSVG mutant 3 and the VSVG mutant 4 1 lentivirus whose viral envelope contains VSVG mutant 4 were packaged separately;
the control VSVG lentivirus 1, VSVG mutant 1 lentivirus, VSVG mutant 2 lentivirus, VSVG mutant 3 lentivirus, VSVG mutant4 lentivirus, VSVG mutant 5 lentivirus, VSVG mutant 6 lentivirus were resuspended respectively in 200 µL of F12 medium, and stored them frozen at -80°C.
HEK-293T cell culture medium: DMEM + 10% FBS;
DMEM: GIBCO, catalog number: #C12430500BT;
FBS: EXCELL, catalog number: #FSP500;
F12 medium: GIBCO, catalog number: #C11330500BT;
Syringe filter: SORFA, catalog number: #622120.

### 4. Viral infection

In another aspect, the designing of the second molecule comprises mutating the amino acid sequence of the second molecule. By designing the first molecule (modified on the viral surface), which are antibodies/ligands capable of targeted recognition of specific receptors, and designing a mutated viral envelope glycoprotein as the second molecule, which has restricted receptor-binding ability but possesses endosomal/lysosomal escape capability and resistance to complement-mediated inactivation, viruses can achieve targeted infection of target cells relying on the first molecule, regardless of whether the target cells express the receptor for the envelope glycoprotein carried by the virus. Meanwhile, the virus will not infect other cells, which greatly improves targeting precision, and it will not be inactivated by complement in the serum.

Day 0, multiple groups of 1×105 Jurkat cells (human T-cell lymphoblastic leukemia cells) were taken respectively, and resuspended them in 200 µL of Jurkat cell culture systems containing complement (10% human plasma) and without complement (10% human serum albumin, HSA) respectively. On the basis of containing 10% human plasma or 10% human albumin, the Jurkat cell culture system comprises 1640 medium (Elgbio, catalog number: EH80809) and 10% FBS.

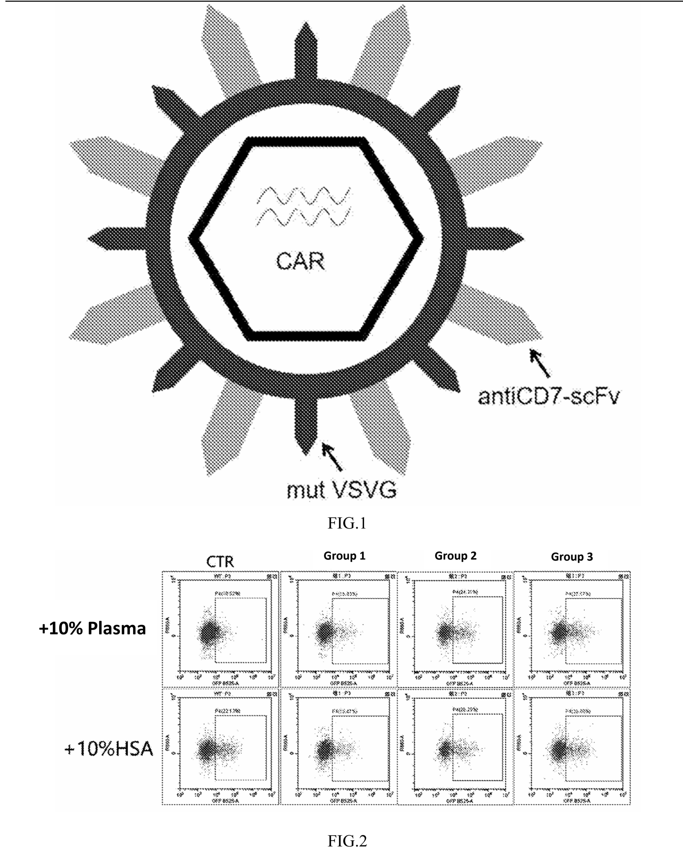

The viral stock solutions of the control VSVG lentivirus 1 VSVG mutant 1 lentivirus, VSVG mutant 3 lentivirus, VSVG mutant 4 lentivirus were concentrated by 200 times respectively. Then 10 µL of the supernatant of each group of viral solutions was taken and added them to each group of the Jurkat cell culture systems containing complement (10% human plasma) or without complement (10% human serum albumin, HSA) respectively, mixed well and placed in a 5% CO2, 37°C incubator for culture. flow cytometry detection was performed on Day 2 to detect the expression of GFP, and the results are shown in FIG.2. The Jurkat cell culture system comprises 1640 medium (brand: Elgbio, catalog number: EH80809) and 10% FBS.

The detection results of the CTR group were the detection results of the infection efficiency of the control VSVG lentivirus 1 infecting CD7+ Jurkat cells in the medium containing human albumin (complement inactivated) and the medium containing human plasma (containing complement), respectively.

The detection results of the group 1 were the detection results of the infection efficiency of the VSVG mutant 1 lentivirus infecting CD7+ Jurkat cells in the medium containing human albumin (complement inactivated) and the medium containing human plasma (containing complement), respectively.

The detection results of the group 2 were the detection results of the infection efficiency of the VSVG mutant 3 lentivirus infecting CD7+ Jurkat cells in the medium containing human albumin (complement inactivated) and the medium containing human plasma (containing complement), respectively.

The detection results of the group 3 were the detection results of the infection efficiency of the VSVG mutant 4 lentivirus infecting CD7+ Jurkat cells in the medium containing human albumin (complement inactivated) and the medium containing human plasma (containing complement), respectively.

As can be seen from FIG.2, in the presence of complement, the control VSVG lentivirus 1, whose viral envelope glycoprotein does not carry the first mutation, is affected by complement inactivation, resulting in a reduced efficiency of infecting CD7+ Jurkat cells (compared to the medium without complement). In contrast, the VSVG mutant 1 lentivirus, VSVG mutant 3 lentivirus, and VSVG mutant 4 lentivirus constructed with VSVG mutants 1, VSVG mutant 3, and VSVG mutant 4 whose viral envelope glycoproteins contain both the first mutation and the second mutation can relatively avoid the impact of complement inactivation on the efficiency of infecting CD7+ Jurkat cells in the presence of complement.

### Example 2

VSVG mutant lentivirus packaging:
control VSVG mutant 2, VSVG mutant 5, VSVG mutant 6 and VSVG mutant 7 were constructed.

Compared with the envelope glycoprotein of the wild-type Vesiculovirus Cocal strain (Cocal-VSVG), the extracellular domains of the VSVG mutant 5, VSVG mutant 6 and VSVG mutant 7 have the first mutation and the second mutation.

The extracellular domain of the envelope glycoprotein of the wild-type Vesiculovirus Cocal strain comprises the amino acid sequence as set forth in SEQ ID NO: 10.

The full-length protein of the envelope glycoprotein of the wild-type Vesiculovirus Cocal strain comprises the amino acid sequence as set forth in SEQ ID NO: 27.

Control VSVG mutant 2 (CTR-VSVG2): the extracellular domain of the envelope glycoprotein of the CTR-VSVG1 comprises the amino acid sequence as set forth in SEQ ID NO: 28. The amino acid sequence as set forth in SEQ ID NO: 28 is the amino acid sequence of SEQ ID NO: 10 with the amino acid at position 354 substituted from arginine (R) to glutamine (Q); that is, compared with the wild-type Cocal-VSVG, the extracellular domain of CTR-VSVG2 has an R354Q mutation.

VSVG mutant 5: the extracellular domain of the envelope glycoprotein of the VSVG mutant 5 comprises the amino acid sequence as set forth in SEQ ID NO: 18. The amino acid sequence as set forth in SEQ ID NO: 18 is the amino acid sequence of SEQ ID NO: 10 with the lysine (K) at position 47 deleted, the amino acid at position 214 (relative to the state before the K47 deletion) substituted from lysine (K) to asparagine (N) and the amino acid at position 352 (relative to the state before the K47 deletion) substituted from threonine (T) to alanine (A); that is, compared with the wild-type Cocal-VSVG, the extracellular domain of VSVG mutant 5 has K47 deletion, K214N mutations and T352A mutations.

VSVG mutant 6: the extracellular domain of the envelope glycoprotein of the VSVG mutant 6 comprises the amino acid sequence as set forth in SEQ ID NO: 20. The amino acid sequence as set forth in SEQ ID NO: 20 is the amino acid sequence of SEQ ID NO: 10 with the amino acid at position 354 substituted from arginine (R) to glutamine (Q), the amino acid at position 214 substituted from lysine (K) to asparagine (N) and the amino acid at position 352 substituted from threonine (T) to alanine (A); that is, compared with the wild-type Cocal-VSVG, the extracellular domain of VSVG mutant 6 has R354Q, K214N and T352A mutations.

With reference to the packaging method for packaging the VSVG mutant 1 lentivirus, VSVG mutant 3 lentivirus and VSVG mutant 4 lentivirus described in Example 1, the control VSVG lentivirus 2 whose viral envelope contains the control VSVG mutant 2; the VSVG mutant 5 lentivirus whose viral envelope contains VSVG mutant 5 and the VSVG mutant 6 lentivirus whose viral envelope contains VSVG mutant 6 were separately packaged.

CD7+ Jurkat cells were infected with the control VSVG lentivirus 2, VSVG mutant 5 lentivirus, and VSVG mutant 6 lentivirus, respectively.

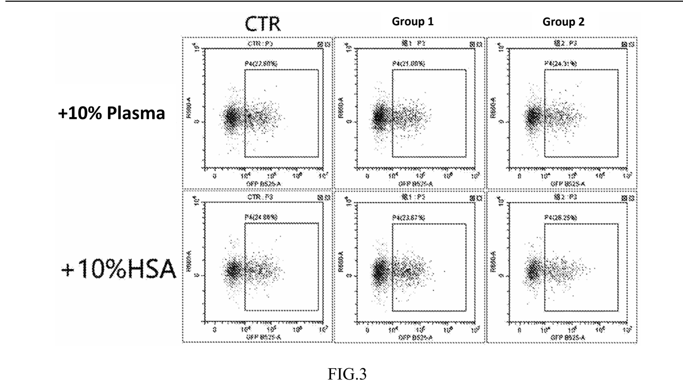

With reference to the method for infecting CD7+ Jurkat cells with VSVG mutant 1 lentivirus, VSVG mutant 3 lentivirus and VSVG mutant 4 lentivirus described in Example 1, the control VSVG lentivirus 2, VSVG mutant 5 lentivirus, and VSVG mutant 6 lentivirus were added respectively to the Jurkat cell culture systems containing complement (10% human plasma) or without complement (10% human serum albumin, HSA) to infect CD7+ Jurkat cells. Perform flow cytometry detection on Day 2 to detect the expression of GFP, and the results are shown in FIG.3;

The detection results of the CTR group are the detection results of the infection efficiency of the control VSVG lentivirus 2 infecting CD7+ Jurkat cells in the medium containing human albumin (complement inactivated) and the medium containing human plasma (containing complement), respectively.

The detection results of the group 1 are the detection results of the infection efficiency of the VSVG mutant 6 lentivirus infecting CD7+ Jurkat cells in the medium containing human albumin (complement inactivated) and the medium containing human plasma (containing complement), respectively.

The detection results of the group1 are the detection results of the infection efficiency of the VSVG mutant 5 lentivirus infecting CD7+ Jurkat cells in the medium containing human albumin (complement inactivated) and the medium containing human plasma (containing complement), respectively.

As can be seen from FIG. 3, the infection efficiency of the VSVG mutant 5 lentivirus with the first mutation K214N+T352A and the second mutation (K47 deletion) is superior to that of the control VSVG lentivirus 2 without the complement inactivation mutation and the VSVG mutant 6 lentivirus with the first mutation K214N+T352A and the second mutation R354Q.

The foregoing are merely specific embodiments of the present invention, but the protection scope of the present invention is not limited thereto. Any change or substitution that can be easily conceived by those skilled in the art within the technical scope disclosed by the present invention shall be covered by the protection scope of the present invention. Therefore, the protection scope of the present invention shall be subject to the protection scope of the claims.

## Claims

1. An envelope glycoprotein of vesicular stomatitis virus with a first mutation and a second mutation, the first mutation causes the envelope glycoprotein to be unable to mediate complement inactivation, the second mutation weakens an ability of the envelope glycoprotein to be recognized by a receptor.

2. The envelope glycoprotein according to claim 1, **characterized in that**, the first mutation is that an extracellular domain of the envelope glycoprotein comprises an amino acid sequence in SEQ ID NO: 1 or an amino acid sequence having at least 50% identity to the amino acid sequence in SEQ ID NO: 1, and the amino acid sequence comprises at least one mutated amino acid as follows:
a) an amino acid at position 214 of SEQ ID NO: 1;
b) an amino acid located at a position corresponding to the position 214 of SEQ ID NO: 1 by best global alignment;
c) an amino acid at position 352 of SEQ ID NO: 1;
d) an amino acid located at a position corresponding to the position 352 of SEQ ID NO: 1 by best global alignment;
e) an amino acid at position 50 of SEQ ID NO: 1;
f) an amino acid located at a position corresponding to the position 50 of SEQ ID NO: 1 by best global alignment;
g) an amino acid at position 146 of SEQ ID NO: 1; or
h) an amino acid located at a position corresponding to the position 146 of SEQ ID NO: 1 by best global alignment.

3. The envelope glycoprotein according to claim 2, **characterized in that**, the mutation is an insertion, deletion or substitution.

4. The envelope glycoprotein according to claim 3, **characterized in that**, the amino acid sequence comprises at least one substituted amino acid as following:
a) the amino acid at position 214 of SEQ ID NO: 1;
b) the amino acid located at a position corresponding to the position 214 of SEQ ID NO: 1 by best global alignment;
c) the amino acid at position 352 of SEQ ID NO: 1;
d) the amino acid located at a position corresponding to the position 352 of SEQ ID NO: 1 by best global alignment;
e) the amino acid at position 50 of SEQ ID NO: 1;
f) the amino acid located at a position corresponding to the position 50 of SEQ ID NO: 1 by best global alignment ;
g) the amino acid at position 146 of SEQ ID NO: 1;
h) the amino acid located at a position corresponding to the position 146 of SEQ ID NO: 1 by best global alignment .

5. The envelope glycoprotein according to claim 4, **characterized in that**, the amino acid sequence comprises one or more mutated amino acid as following: substitution of the amino acid at position T214, substitution of the amino acid at position T352, substitution of the amino acid at position K50, substitution of the amino acid at position S146.

6. The envelope glycoprotein according to claim 5, **characterized in that**, the amino acid sequence comprises one or more mutated amino acid as follows: the amino acid at position 214 substituted from threonine (T) to asparagine (N), the amino acid at position 352 substituted from threonine (T) to alanine (A), the amino acid at position 50 substituted from lysine (K) to threonine (T), or the amino acid at position 146 substituted from serine (S) to threonine (T).

7. The envelope glycoprotein according to claim 4, **characterized in that**, the amino acid sequence comprises one of mutated amino acid combinations as following:
(1) substitution of the amino acid at position K214 and T352;
(2) substitution of the amino acid at position K214, T352, K50, and S146.

8. The envelope glycoprotein according to claim 7, **characterized in that**, the amino acid sequence comprises one of mutated amino acid combinations as follows:
(1) the amino acid at position 214 substituted from lysine (K) to asparagine (N) and the amino acid at position 352 substituted from threonine (T) to alanine (A);
(2) the amino acid at position 214 substituted from lysine (K) to asparagine (N), the amino acid at position 352 substituted from threonine (T) to alanine (A), the amino acid at position 50 substituted from lysine (K) to threonine (T) and the amino acid at position 146 substituted from serine (S) to threonine (T).

9. The envelope glycoprotein according to claim 6, **characterized in that**, the amino acid sequence comprises one of mutated amino acid combinations as follows:
(1) the amino acid at position 214 substituted from threonine (T) to asparagine (N) and the amino acid at position 352 substituted from threonine (T) to alanine (A); or
(2) the amino acid at position 214 substituted from threonine (T) to asparagine (N), the amino acid at position 352 substituted from threonine (T) to alanine (A), the amino acid at position 50 substituted from lysine (K) to threonine (T) and the amino acid at position 146 substituted from serine (S) to threonine (T)

10. The envelope glycoprotein according to any one of claims 1 to 9, **characterized in that**, the second mutation is that an extracellular domain of the envelope glycoprotein comprising an amino acid sequence in SEQ ID NO: 1 or an amino acid sequence having at least 50% identity to the amino acid sequence in SEQ ID NO: 1, and the amino acid sequence comprises at least one mutated amino acid as follows:
1) substitution/deletion of an amino acid at position H8, substitution/deletion of an amino acid at position N9, substitution/deletion of an amino acid at position Q10, substitution/deletion of an amino acid at position K47, substitution/deletion of an amino acid at position K50, substitution/deletion of an amino acid at position A51, substitution/deletion of an amino acid at position S183, substitution/deletion of an amino acid at position S179, substitution/deletion of an amino acid at position N180, substitution/deletion of an amino acid at position I182, substitution/deletion of an amino acid at position M184, substitution/deletion of an amino acid at position Y209, substitution/deletion of an amino acid at position I347, substitution/deletion of an amino acid at position T350, substitution/deletion of an amino acid at position T352, substitution/deletion of an amino acid at position E353, substitution/deletion of an amino acid at position R354, deletion of amino acids at positions 1-18, deletion of amino acids at positions 19-36, deletion of amino acids at positions 37-51, deletion of amino acids at positions 314-384, deletion of amino acids at positions 321-374, deletion of amino acids at positions 331-364, deletion of amino acids at positions 344-354, and deletion of amino acids at positions 345-353 of SEQ ID NO: 1; or
2) substitution/deletion of an amino acid at a position corresponding to the position H8, substitution/deletion of an amino acid at a position corresponding to the position N9, substitution/deletion of an amino acid at a position corresponding to the position Q10, substitution/deletion of an amino acid at a position corresponding to the position K47, substitution/deletion of an amino acid at a position corresponding to the position K50, substitution/deletion of an amino acid at a position corresponding to the position A51, substitution/deletion of an amino acid at a position corresponding to the position S183, substitution/deletion of an amino acid at a position corresponding to the position S179, substitution/deletion of an amino acid at a position corresponding to the position N180, substitution/deletion of an amino acid at a position corresponding to the position I182, substitution/deletion of an amino acid at a position corresponding to the position M184, substitution/deletion of an amino acid at a position corresponding to the position Y209, substitution/deletion of an amino acid at a position corresponding to the position I347, substitution/deletion of an amino acid at a position corresponding to the position T350, substitution/deletion of an amino acid at a position corresponding to the position T352, substitution/deletion of an amino acid at a position corresponding to the position E353, substitution/deletion of an amino acid at a position corresponding to the position R354, deletion of amino acids at positions corresponding to the positions 1-18, deletion of amino acids at positions corresponding to the positions 19-36, deletion of amino acids at positions corresponding to the positions 37-51, deletion of amino acids at positions corresponding to the positions 314-384, deletion of amino acids at positions corresponding to the positions 321-374, deletion of amino acids at positions corresponding to the positions 331-364, deletion of amino acids at positions corresponding to the positions 344-354, and deletion of amino acids at positions corresponding to the positions 345-353 of SEQ ID NO: 1 by best global alignment.

11. The envelope glycoprotein according to claim 10, **characterized in that**, the amino acid sequence comprises at least one mutated amino acid as follows:
1) substitution of the amino acid at position H8, substitution of the amino acid at position N9, substitution of the amino acid at position Q10, substitution of the amino acid at position K47, deletion of the amino acid at position K47, substitution of the amino acid at position K50, substitution of the amino acid at position A51, substitution of the amino acid at position S183; substitution of the amino acid at position S179; substitution of the amino acid at position N180, substitution of the amino acid at position I182, substitution of the amino acid at position M184, substitution of the amino acid at position Y209, substitution of the amino acid at position I347, substitution of the amino acid at position T350, substitution of the amino acid at position T352, substitution of the amino acid at position E353, substitution of the amino acid at position R354, deletion of the amino acid at position R354, deletion of the amino acids at positions 1-18, deletion of the amino acids at positions 19-36, deletion of the amino acids at positions 37-51, deletion of the amino acids at positions 314-384, deletion of the amino acids at positions 321-374, deletion of the amino acids at positions 331-364, deletion of the amino acids at positions 344-354, deletion of the amino acids at positions 345-353 of SEQ ID NO: 1;
2) substitution of the amino acid at the position corresponding to the position H8, substitution of the amino acid at the position corresponding to the position N9, substitution of the amino acid at the position corresponding to the position Q10, substitution of the amino acid at the position corresponding to the position K47, deletion of the amino acid at the position corresponding to the position K47, substitution of the amino acid at the position corresponding to the position K50, substitution of the amino acid at the position corresponding to the position A51, substitution of the amino acid at the position corresponding to the position S183; substitution of the amino acid at the position corresponding to the position S179; substitution of the amino acid at the position corresponding to the position N180, substitution of the amino acid at the position corresponding to the position I182, substitution of the amino acid at the position corresponding to the position M184, substitution of the amino acid at the position corresponding to the position Y209, substitution of the amino acid at the position corresponding to the position I347, substitution of the amino acid at the position corresponding to the position T350, substitution of the amino acid at the position corresponding to the position T352, substitution of the amino acid at the position corresponding to the position E353, substitution of the amino acid at the position corresponding to the position R354, deletion of the amino acid at the position corresponding to the position R354, deletion of the amino acids at the positions corresponding to the positions 1-18, deletion of the amino acids at the positions corresponding to the positions 19-36, deletion of the amino acids at the positions corresponding to the positions 37-51, deletion of the amino acids at the positions corresponding to the positions 314-384, deletion of the amino acids at the positions corresponding to the positions 321-374, deletion of the amino acids at the positions corresponding to the positions 331-364, deletion of the amino acids at the positions corresponding to the positions 344-354, deletion of the amino acids at the positions corresponding to the positions 345-353 of SEQ ID NO: 1 by best global alignment.

12. The envelope glycoprotein according to claim 11, **characterized in that**, the amino acid sequence comprises one or more mutated amino acid as follows: substitution/deletion of the amino acid at position K47, substitution/deletion of the amino acid at position R354.

13. The envelope glycoprotein according to claim 12, **characterized in that**, the amino acid sequence comprises one or more mutated amino acid as follows: the amino acid at position 47 substituted from lysine (K) to glutamine (Q), the amino acid at position 354 substituted from arginine (R) to glutamine (Q), deletion of the amino acid at position 47, or deletion of the amino acid at position 354.

14. The envelope glycoprotein according to claim 12, **characterized in that**, the amino acid sequence comprises one of the mutated amino acid combinations as follows:
(1) substitution of the amino acid at position K47;
(2) substitution of the amino acid at position R354.

15. The envelope glycoprotein according to claim 14, **characterized in that**, the amino acid sequence comprises one of the mutated amino acid combinations as follows:
(1) the amino acid at position 47 substituted from lysine (K) to glutamine (Q);
(2) the amino acid at position 354 substituted from arginine (R) to glutamine (Q).

16. The envelope glycoprotein according to any one of claims 1 to 9, **characterized in that**, the second mutation is that an extracellular domain of the envelope glycoprotein comprises an amino acid sequence in SEQ ID NO: 10, and the amino acid sequence comprises at least one mutated amino acid as follows:
substitution/deletion of an amino acid at position Q8, substitution/deletion of an amino acid at position S9, substitution/deletion of an amino acid at position Q10, substitution/deletion of an amino acid at position K47, substitution/deletion of an amino acid at position K50, substitution/deletion of an amino acid at position A51, substitution/deletion of an amino acid at position D183, substitution/deletion of an amino acid at position A179, substitution/deletion of an amino acid at position T180, substitution/deletion of an amino acid at position V182, substitution/deletion of an amino acid at position T 184, substitution/deletion of an amino acid at position Y209, substitution/deletion of an amino acid at position I347, substitution/deletion of an amino acid at position S350, substitution/deletion of an amino acid at position T352, substitution/deletion of an amino acid at position E353, substitution/deletion of an amino acid at position R354, deletion of amino acids at positions 1-18, deletion of amino acids at positions 19-36, deletion of amino acids at positions 37-51, deletion of amino acids at positions 314-384, deletion of amino acids at positions 321-374, deletion of amino acids at positions 331-364, deletion of amino acids at positions 344-354, and deletion of amino acids at positions 345-353 of SEQ ID NO: 10; or
substitution/deletion of an amino acid at a position corresponding to the position Q8, substitution/deletion of an amino acid at a position corresponding to the position S9, substitution/deletion of an amino acid at a position corresponding to the position Q10, substitution/deletion of an amino acid at a position corresponding to the position K47, substitution/deletion of an amino acid at a position corresponding to the position K50, substitution/deletion of an amino acid at a position corresponding to the position A51, substitution/deletion of an amino acid at a position corresponding to the position D183, substitution/deletion of an amino acid at a position corresponding to the position A179, substitution/deletion of an amino acid at a position corresponding to the position T180, substitution/deletion of an amino acid at a position corresponding to the position V182, substitution/deletion of an amino acid at a position corresponding to the position T184, substitution/deletion of an amino acid at a position corresponding to the position Y209, substitution/deletion of an amino acid at a position corresponding to the position I347, substitution/deletion of an amino acid at a position corresponding to the position S350, substitution/deletion of an amino acid at a position corresponding to the position T352, substitution/deletion of an amino acid at a position corresponding to the position E353, substitution/deletion of an amino acid at a position corresponding to the position R354, deletion of amino acids at positions corresponding to the positions 1-18, deletion of amino acids at positions corresponding to the positions 19-36, deletion of amino acids at positions corresponding to the positions 37-51, deletion of amino acids at positions corresponding to the positions 314-384, deletion of amino acids at positions corresponding to the positions 321-374, deletion of amino acids at positions corresponding to the positions 331-364, deletion of amino acids at positions corresponding to the positions 344-354, and deletion of amino acids at positions corresponding to the positions 345-353 of SEQ ID NO: 10 by best global alignment.

17. The envelope glycoprotein according to claim 16, **characterized in that**, the amino acid sequence comprises at least one mutated amino acid as follows:
substitution of the amino acid at position Q8, substitution of the amino acid at position S9, substitution of the amino acid at position Q10, substitution or deletion of the amino acid at position K47, substitution of the amino acid at position K50, substitution of the amino acid at position A51, substitution of the amino acid at position D183, substitution of the amino acid at position A179, substitution of the amino acid at position T180, substitution of the amino acid at position V182, substitution of the amino acid at position T184, substitution of the amino acid at position Y209, substitution of the amino acid at position I347, substitution of the amino acid at position S350, substitution of the amino acid at position T352, substitution of the amino acid at position E353, substitution or deletion of the amino acid at position R354, deletion of the amino acids at positions 1-18, deletion of the amino acids at positions 19-36, deletion of the amino acids at positions 37-51, deletion of the amino acids at positions 314-384, deletion of the amino acids at positions 321-374, deletion of the amino acids at positions 331-364, deletion of the amino acids at positions 344-354, deletion of the amino acids at positions 345-353 of SEQ ID NO: 10;
substitution of the amino acid at the position corresponding to the position Q8, substitution of the amino acid at the position corresponding to the position S9, substitution of the amino acid at the position corresponding to the position Q10, substitution or deletion of the amino acid at the position corresponding to the position K47, substitution of the amino acid at the position corresponding to the position K50, substitution of the amino acid at the position corresponding to the position A51, substitution of the amino acid at the position corresponding to the position D183, substitution of the amino acid at the position corresponding to the position A179, substitution of the amino acid at the position corresponding to the position T180, substitution of the amino acid at the position corresponding to the position V182, substitution of the amino acid at the position corresponding to the position T184, substitution of the amino acid at the position corresponding to the position Y209, substitution of the amino acid at the position corresponding to the position I347, substitution of the amino acid at the position corresponding to the position S350, substitution of the amino acid at the position corresponding to the position T352, substitution of the amino acid at the position corresponding to the position E353, substitution or deletion of the amino acid at the position corresponding to the position R354, deletion of the amino acids at the positions corresponding to the positions 1-18, deletion of the amino acids at the positions corresponding to the positions 19-36, deletion of the amino acids at the positions corresponding to the positions 37-51, deletion of the amino acids at the positions corresponding to the positions 314-384, deletion of the amino acids at the positions corresponding to the positions 321-374, deletion of the amino acids at the positions corresponding to the positions 331-364, deletion of the amino acids at the positions corresponding to the positions 344-354, deletion of the amino acids at the positions corresponding to the positions 345-353 of SEQ ID NO: 10 by best global alignment.

18. The envelope glycoprotein according to claim 17, **characterized in that**, the amino acid sequence comprises one or more mutated amino acid as follows: substitution/deletion of the amino acid at position K47, substitution/deletion of the amino acid at position R354.

19. The envelope glycoprotein according to claim 18, **characterized in that**, the amino acid sequence comprises one or more mutated amino acid as follows: the amino acid at position 47 substituted from lysine (K) to glutamine (Q), the amino acid at position 354 substituted from arginine (R) to glutamine (Q), deletion of the amino acid at position 47, or deletion of the amino acid at position 354.

20. The envelope glycoprotein according to any one of claims 1 to 19, **characterized in that**, the receptor is LDL-R.

21. The envelope glycoprotein according to any one of claims 1 to 20, **characterized in that**, an extracellular domain of the envelope glycoprotein comprises an amino acid sequence in any one of SEQ ID NO: 2, SEQ ID NO: 3, SEQ ID NO: 11, SEQ ID NO: 12, SEQ ID NO: 17, SEQ ID NO: 18, SEQ ID NO: 19 or SEQ ID NO: 20.

22. A nucleic acid molecule, encoding the envelope glycoprotein according to any one of claims 1 to 21.

23. A vector, comprising at least one nucleic acid molecule according to claim 22, or expressing the envelope glycoprotein according to any one of claims 1 to 21.

24. The vector according to claim 23, **characterized in that**, the vector is a lentiviral vector or a retroviral vector.

25. A targeting vector, comprising: a first molecule that binds to an endocytic receptor of target cell and a second molecule that is non complement inactivated and promotes a release of substance carried by the targeting vector into cytoplasm.

26. The targeting vector according to claim 25, **characterized in that**, the second molecule promotes endosomal escape or lysosomal escape of the targeting vector, and prevents the targeting vector from being inactivated by complement.

27. The targeting vector according to claim 26, **characterized in that**, the second molecule is the envelope glycoprotein according to any one of claims 1 to 21.

28. The targeting vector according to any one of claims 25 to 27, **characterized in that**, the targeting vector is an enveloped virus.

29. The targeting vector according to claim 28, **characterized in that**, the enveloped virus is selected from a retroviral vector or a lentiviral vector.

30. The targeting vector according to claim 29, **characterized in that**, the lentiviral vector is a VSV vector.

31. The targeting vector according to claim 29 or 30, **characterized in that**, the first molecule is not a part of a viral envelope glycoprotein.

32. The targeting vector according to any one of claims 25 to 31, **characterized in that**, the first molecule comprises a transmembrane peptide segment, an antibody or ligand that binds to the endocytic receptor of the target cell.

33. The targeting vector according to claim 32, **characterized in that**, the first molecule further comprises an extracellular hinge region.

34. The targeting vector according to claim32 or 33, **characterized in that**, the endocytic receptor is selected from: HER2, CD20, CD19, CD79A, CD79B, CD56, CD22, CD138, CD37, CD98, CD309, CD33, CD163, CD163B, CD5, CD7, CD169, CD204, CD205, CD209, CD280, CD302, TROP-2, CD19, NECTIN4, 5T4, CD30, TROP2, FRα, STEAP1, ENPP3, GCC, SLC44A4, NaPi2b, CA9, SC-16, CD142, P-Cadherin, PSMA, ED-B, endothelin ETB, TN-C, Collagen IV, Periostin, CEACAM, c-MET, TDGF1, IGF1R, Mesothelin, TIM1, NCAM1, ZIP6, CD166, GPNMB, SDC1, glycosphingolipid, TfR, Ganglioside, CD74, CLDN18, DPEP3, SLITRK6, PRL-R, LY75, CD48, MUC1, CDKs, B7-H4, STING, KAAG1, CD70, CDH3, LRRC15, EGFR or ASGPR.

35. The targeting vector according to claim 34, **characterized in that**, the endocytic receptor is CD7.

36. The targeting vector according to claim 35, **characterized in that**, the antibody is anti-CD7 single-domain antibody, an HCDR1 region of the anti-CD7 single-domain antibody comprises an amino acid sequence in SEQ ID NO:22, or an amino acid sequence having at least 75%, 80%, 90%, 91%, 92%, 93%, 94%, 95%, 96%, 97%, 98% or 99% identity to the amino acid sequence in SEQ ID NO:22; an HCDR2 region of the anti-CD7 single-domain antibody comprises an amino acid sequence in SEQ ID NO:23, or an amino acid sequence having at least 75%, 80%, 90%, 91%, 92%, 93%, 94%, 95%, 96%, 97%, 98% or 99% identity to the amino acid sequence in SEQ ID NO:23; an HCDR3 region of the anti-CD7 single-domain antibody comprises an amino acid sequence in SEQ ID NO:24, or an amino acid sequence having at least 75%, 80%, 90%, 91%, 92%, 93%, 94%, 95%, 96%, 97%, 98% or 99% identity to the amino acid sequence in SEQ ID NO:24.

37. The targeting vector according to claim 36, **characterized in that**, the anti-CD7 single-domain antibody (VHH) comprises an amino acid sequence in SEQ ID NO:21, or an amino acid sequence having at least 75%, 80%, 90%, 91%, 92%, 93%, 94%, 95%, 96%, 97%, 98% or 99% identity to the amino acid sequence in SEQ ID NO:21.

38. The targeting vector according to any one of claims 25-37, **characterized in that**, the substance is at least one of small molecule compound, protein, polypeptide, RNA or DNA.

39. The targeting vector according to any one of claims 38, **characterized in that**, the substance comprises polynucleotides encoding chimeric antigen receptor.

40. The targeting vector according to any one of claims 25 to 39, **characterized in that**, the target cell is lymphocyte, myeloid cell, hematopoietic stem/progenitor cell or non-blood cell.

41. A preparation method of the targeting vector according to any one of claims 25 to 40, comprising the following steps:
designing the first molecule and designing the second molecule according to the endocytic receptor of the target cell,
assembling the first molecule and the second molecule with the substance carried by a vector to prepare the targeting vector.

42. The preparation method according to claim 41, **characterized in that**, designing the second molecule comprises mutating an amino acid sequence of the second molecule.

43. Use of the targeting vector according to any one of claims 25 to 40 in drug delivery or vaccine delivery.

44. Use of the targeting vector according to any one of claims 25 to 40 for delivering small molecule compounds, proteins, polypeptides, RNA or DNA.

45. A method of introducing the substance into a cell, comprising: contacting the cell with the targeting vector according to any one of claims 25 to 40.

46. The method according to claim 45, **characterized in that**, the cell is mammalian cell.

47. The method according to claim 45, **characterized in that**, the cell is normal cell or cancer cell.

48. The method according to claim 45, **characterized in that**, the cell is T cell, NK cell, B cell, macrophage, granulocyte, dendritic cell, hematopoietic stem cell, hepatocyte, islet cell, neuron or muscle cell.

49. The method according to claim 45, **characterized in that**, the contacting is performed in vivo or in vitro.

50. A host cell, comprising or expressing the envelope glycoprotein according to any one of claims 1 to 21, or comprising the nucleic acid molecule according to claim 22, or comprising the targeting vector according to any one of claims 25 to 40.

51. A composition, comprising the envelope glycoprotein according to any one of claims 1 to 21, or comprising the nucleic acid molecule according to claim 22, or the targeting vector according to any one of claims 25 to 40, or the host cell according to claim 50, or any combination thereof.

52. The composition according to claim 51 for use as a medicament.

53. The composition according to claim 51 for use in gene therapy, immunotherapy, cell therapy, treatment of genetic deficiency diseases, or treatment of cancer.

54. A method for treating a disease suffered by a subject or killing disease cells in a subject, comprising administering the composition according to any one of claims 51 to 53 to the subject.

55. The method according to claim 54, **characterized in that**, the disease comprises cancer, and the cancer comprises hematological cancer and solid cancer.

56. The method according to claims 54 or 55, **characterized in that**, the administrating is selected from at least one of administration via oral, nasal, intravenous, intraperitoneal, intracerebral (intraparenchymal), intraventricular, intramuscular, intraocular, intraarterial, portal vein, intralesional, a sustained release system, or an implantable device.
